(19) **Europäisches Patentamt / European Patent Office / Office européen des brevets**

(11) **EP 2 278 508 A1**

(12) **EUROPEAN PATENT APPLICATION**

(43) Date of publication:
26.01.2011 Bulletin 2011/04

(51) Int Cl.:
***G06F 19/00*** (2011.01)

(21) Application number: 10169739.9

(22) Date of filing: 15.07.2010

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: 17.07.2009 GB 0912462

(71) Applicant: **Sharp Kabushiki Kaisha**
**Osaka 545-8522 (JP)**

(72) Inventors:
• **Dothie, Pamela Ann**
 **Oxford, Oxfordshire OX4 4GB (GB)**
• **Ford, Thomas Alexander**
 **Oxford, Oxfordshire OX4 4GB (GB)**

(74) Representative: **Suckling, Andrew Michael**
**Marks & Clerk LLP**
**Oxford Business Park South**
**Oxford, Oxfordshire, OX4 2RU (GB)**

(54) **Method and system for managing a user's sleep**

(57)    A sleep management method and system for improving the quality of sleep of a user which monitors one or more objective parameters relevant to sleep quality of the user when in bed and receives from the user in waking hours via a portable device such as a mobile phone feedback from objective test data on cognitive and / or psychomotor performance.

FIG. 1

EP 2 278 508 A1

(Cont. next page)

FIG. 2

| Lifestyle Data |
| --- |
| Sleep questionnaires<br>Diet and exercise<br>Perceived stress |

| Cognitive Data |
| --- |
| Reaction time tests<br>Logical reasoning<br>Mathematical tests |

| Portable Sensor Data |
| --- |
| Temperature<br>Noise<br>Accelerometer |

Screen

User input keypad

Speaker

Battery

Vibrate unit

Memory

Network connection

**Description**

**FIELD OF THE INVENTION**

The present invention relates to a system and method for managing a user's sleep, for example with a view to improving sleep quality of the user. Such a system and method may be used for monitoring and analysing sleep information, for example for monitoring objective sleep related parameters measured whilst the user is sleeping and correlating that information with objective and subjective sleep related parameters measured whilst the user is awake.

**BACKGROUND TO THE INVENTION**

Sleeplessness and fatigue are major unaddressed problems in the developed world. A significant proportion of the population experiences problems sleeping at some point in their lives. Lack of sleep is linked to a poorer quality of life, reduced performance at work and, in more severe cases, to mental disorders such as depression. For drivers, or those who work in industries where there is a risk of accident, sleepiness can contribute to the frequency of serious accidents. There is therefore a large potential market for devices aimed at improving the quality of consumers' sleep.

There is little evidence that poor sleep or sleep deprivation results in immediate physiological damage, but it can impact on an individual's quality of life. The following list indicates some of the consequences of poor sleep:

- Significant reductions in performance and alertness
- Impaired memory and cognitive ability
- Disruption of bed partner's sleep
- Greater risk of sustaining an occupational injury
- Greater risk of being involved in a motor vehicle accident
- Disturbances in metabolism
- Psychiatric effects
- Problems with the immune system
- Poorer quality of life
- Reduced feeling of wellbeing
- Increased risk of depression

Sleep is a dynamic behaviour, known to be made up of four non-rapid eye movement (NREM) stages (I-IV) and one rapid eye movement (REM) stage. Sleep stages I and II are associated with light sleep while sleep stages III and IV are associated with deep sleep. Non-REM sleep is usually associated with minimal mental activity, whereas REM sleep is associated with a highly active brain and dreaming. For a typical adult, sleep progresses through stages I, II, III and IV before reversing itself back through stages III to I and then to REM sleep. This cycle is repeated at approximately 90-100 minute intervals throughout the night. Deep sleep is more prevalent in the early part of the night, with sleep becoming gradually lighter in the later parts of the night.

Body movements whilst sleeping are normal and are typically associated with light sleep (stages I and II); a lack of body movements is typically indicative of deep sleep stages (III and IV). Men tend to have significantly more discrete movements than women during the night while younger people tend to have more discrete movements during sleep than older people. The monitoring of movement may also provide an indication of restlessness which may lead to poor sleep quality. This may be due to an inability to sleep, nightmares, illness or other causes.

There are several environmental parameters that can have an effect on sleep structure. Temperature is found to have a significant effect on the quality of sleep. Both hot and cold temperatures can have a detrimental impact on the quality of sleep with cold room temperatures (for example, below 21°C) affecting sleep structure more than warmer room temperatures (for example, above 27°C). In these instances, total sleep duration, REM sleep and stage IV sleep are reduced compared to sleeping in a room at a comfortable temperature. This is significant because during REM sleep, the body can not regulate its temperature. It follows that the waking-sleeping cycle is affected by ambient temperature.

High humidity can also have a detrimental effect on the structure of sleep and sleep quality, particularly when combined with high room temperatures.

Noise can also impact on sleep quality. The absence or presence of familiar noise can have as great an impact on sleep as out-of-the ordinary noises and people often adapt to accept familiar noises without any impact on sleep. For example, people who live in close proximity to airports are rarely awakened specifically by aircraft noise. In general, older people tend to be more affected by noise disturbances than younger people. Noise disturbed sleep is also usually accompanied by an increase in limb/body movements.

The circadian rhythm is an internal body clock which has a period of about a day and is controlled by the suprachiasmatic nucleus (SCN) of the anterior hypothalamus of the brain. Circadian rhythm, which influences when we feel sleepy and alert, can be affected by sleep/wake schedules, rest/activity and changes in body temperature. Circadian rhythms can also be characterized by melatonin secretion by the pineal gland, which peaks at night and fades during the day. Cortisol levels in blood can also vary as a function of circadian rhythm. Light is a powerful regulator of circadian rhythm and biological clocks, particularly blue light of the wavelength 450-480nm.

Exogenous and endogenous factors can influence circadian rhythms. Endogenous factors influencing sleep arise as a result of circadian and homeostatic drivers regulated by internal body clocks. Exogenous factors affecting sleep arise as a result of lifestyle or environmental parameters. Homeostatic drivers for sleep represent the increased need for sleep the longer one is awake.

Core body temperature varies as a function of circadian rhythm. A human who follows a nocturnal sleep and diurnal wake sleeping pattern, sleeping for 8 hours and being awake for 16 hours, might find that their core temperature peaks between the hours of 19:00 and 23:00, not long before sleep onset, and reaches a minimum between 03:00 and 06:00 whilst asleep. Core body temperature generally increases gradually whilst the user is awake back up to its peak value of approximately 37-37.6°C. Some people experience a small dip in body temperature in the afternoon, typically between the hours of 14:00 and 16:00. However, not everyone experiences this mid-afternoon lull. Core body temperature has been well correlated to performance with higher core body temperatures generally correlating with better performance and lower core body temperatures generally correlating with poorer performance.

Individuals often exhibit differences in their circadian amplitude and circadian phase. Some people perform consistently better in the morning whilst others perform consistently better in the evening; this is a direct result of endogenous differences in the circadian rhythm of individual biological clocks. Circadian amplitude refers to the range of values that can be assumed over the course of the circadian cycle, e.g. range of core body temperatures, whereas circadian phase refers to any point on that cycle, e.g. the time the user wakes each day.

Knowledge of circadian rhythm is important as it provides information fundamental to understanding normal and abnormal sleep. It also provides an insight into the biological and physiological functioning of the human body. Furthermore, knowledge of circadian rhythm can help a user understand at what point(s) in the day they reach their optimal functioning ability.

The 'gold standard' method of monitoring sleep, polysomnography, is an invasive procedure using a plurality of electrodes attached to the body in order to record an electroencephalogram (EEG), electro-oculogram (EOG), electromyogram (EMG), electrocardiogram (ECG) and the respiratory movements of the abdomen and thorax. Further sensors can be used to measure blood oxygen saturation levels and nasal airflow. Due to the complexity of the procedure, polysomnography is normally carried out by trained professionals in a supervised sleep laboratory. The procedure is invasive, time consuming and uncomfortable, and can actually prevent the patient sleeping as they would on a normal night.

Actigraphy can also be used to assess certain sleep parameters of a patient by recording their gross body movements using accelerometers. The actigraph, which is the size of a wrist watch, can be worn either on the patient's wrist or ankle. When worn overnight, the actigraph can be used to estimate some sleep parameters, such as time in bed (TIB) and total sleep time (TST). However, it can overestimate the amount of sleep obtained by patients suffering from insomnia as they tend to be very good at lying still for long periods of time. The actigraph is useful for monitoring general levels of daytime activity.

For polysomnography and/or actigraphy assessments, patients are usually requested to fill out a sleep diary for a number of weeks prior to the procedure. Sleep diaries often request the user to detail information such as: what time they went to bed, what time they fell asleep, what time they woke up, what time they got out of bed, how many night time awakenings they had, the times and reasons for the night time awakenings, how long it took them to fall back asleep after awakening, whether they napped during the day and also lifestyle data such as the amount of time spent exercising, the amount of alcohol consumed, the amount of caffeine consumed, perceived stress levels, whether and when any medication was consumed (including sleeping pills) and whether or not a heavy meal was eaten immediately prior to going to bed. There are several problems associated with this approach, not least because the questions are very subjective. It is well known that, for example, patients are not always able to accurately assess how long it took them to fall asleep, when they initially fell asleep, or how long it took them to fall back asleep after a night time awakening; insomniacs frequently overestimate the amount of time they spend lying awake in bed before falling back asleep. As a result, sleep diaries can often be inaccurate. This in turn can then lead to inaccuracies in the parameters in which a sleep professional is interested, such as total sleep time (TST), time in bed (TIB), sleep onset latency (SOL), wake after sleep onset (WASO), number of awakenings after sleep onset (NWAK) and sleep efficiency (SE).

Patients may also be asked to fill in a number of scientifically established questionnaires that determine the patient's perception of their tiredness. The Epworth Sleepiness Scale (ESS) tests how likely the patient is to fall asleep or doze in a number of everyday scenarios in contrast to just feeling tired. Even if the tasks in the questions do not directly apply to the patient, they have to try to establish how they would have been affected if they had been in that situation. The Stanford Sleepiness Scale (SSS) is a quick method for assessing how alert a user feels; most people have two peak times of daily alertness which depend on their circadian rhythm.

Other recognized sleep quality questionnaires include the Functional Outcomes of Sleep questionnaire (FOSQ), Pittsburgh Sleep Quality Index (PSQI), International Restless Legs Syndrome Study Group Questionnaire (IRLSSG), Hospital Anxiety and Depression scale (HADS) and general sleep hygiene questionnaires.

For patients with insomnia, some psychological questionnaires may also be administered; The Beck Depression Inventory (BDI), the Spielberger State-Trait Anxiety Inventories, the Profile of Mood States and the Brief Symptom Inventory.

Further to monitoring perceived tiredness, mental fatigue can also be monitored in order to track sleep quality; certain aspects of complex tasks are affected by sleep loss or deprivation, particularly the ability to think laterally. Mental fatigue relates to decreased performance and alertness which can impair cognitive function and memory. (Alertness is defined to mean selective and sustained attention whilst performance relates to cognitive ability.) Therefore, objective measures of fatigue can be measured using a variety of neurobehavioral assessments, such as cognitive and psychomotor tests.

Cognitive and psychomotor tests may include the Psychomotor Vigilance Task (PVT) which is a well established method in the literature for measuring response times ("Cumulative sleepiness, mood disturbance and psychomotor vigilance performance decrements during a week of sleep restricted to 4-5 hours per night", Dinges et al, Sleep, 20, 267-277, 1997), which vary as a function of alertness, and the Stroop Colour Word Test, which takes advantage of our ability to read words more quickly and automatically than we can name colours.

A detailed list of tasks that can be used to monitor cognitive and psychomotor performance is given in Table 1 (taken from 'Cognitive and psychomotor performance tests and experiment design in multiple chemical sensitivity', Anthony Wetherell, Environmental Health Perspectives, Vol 105, Supplement 2, March 1997).

| Table 1: Cognitive and Psychomotor Performance Tests | |
| --- | --- |
| **Mathematical Processing** | |
| Numerical processing | User has to state whether a series of problems consisting of three digits and two operators is greater or less than a given value. |
| Number facility | User sums a series of three one- or two- digit numbers and inserts the answer. |
| | |
| **Logical Reasoning** | |
| Original version | User is presented with a series of sentences each followed by a pair of letters e.g. AB or BA. The sentence describes the order of the letters, e.g. A follows B and subjects have to say whether the statement is true or false. |
| AGARD STRES version | A series of pairs of sentences each followed be three symbols, e.g. #&*, is presented. The sentences describe the order of the symbols, e.g. & before #, & after * and the subject presses a key signifying whether the sentences are true or false. |
| | |
| **Spatial Processing** | |
| Manikin | A front or back view of a human holding a flag and rotated to any angle. User has to specify which hand is holding the flag. |
| Histograms | A four-bar histogram is presented to a user for 3s followed by a blank screen for 1s. A second histogram is then presented to the user rotated by a certain angle. The subject must state whether the two histograms are the same or different. |
| | |
| **Tracking** | |
| Pursuit tracking | The subject attempts to keep a cursor on a moving target for a specified amount of time. |
| Unstable tracking | The subject attempts to keep a horizontally moving cursor on a fixed target. |
| | |

(continued)

| | |
|---|---|
| **Reaction time** | |
| Simple reaction time | Subject presses a key as quickly as possible after shown a stimulus. |
| Choice reaction time | Subject presses one of several keys as quickly as possible after various stimuli. |
| Complex reaction time | Test based on the stage processing model designed to identify the locus of a drug effect. |
| | |
| **Attention/vigilance** | |
| Letter cancellation | Matrices of random letters are presented to the subject who cross out or mark certain letters. |
| Serial response | A row of 5 outlined squares corresponding to the keys 1-5 on a keyboard. Subjects 'chase' a black square that appears at random in one of the outlined squares by pressing the appropriate key. |
| Focused attention | Three warning crosses are presented, one in the middle of the screen, the other two either close to it or close to the edges of the screen. The middle cross is replaced by a target letter (e.g. A or B) and the other crosses by asterisks, the same letter as the target, or the other letter. The user responds to the target letter by pressing the appropriate key. |
| Search | Two warning crosses are presented close to the middle or close to the edges of the screen. One cross is then replaced by a target letter (e.g. A or B) and the other is either replaced by a digit or disappears. The user responds to the target letter by pressing an appropriate key. |
| Display monitoring | Subjects watch the display of a scale and a moving pointer. At random intervals, the pointer tends to stay in one half of its scale. Subjects must report when this occurs. |
| Vigilance | Several auditory and visual vigilance tests are used all requiring subjects to detect signals or targets in noise. |
| Colour word naming | The names of words are presented in either their own colour or a different colour; the subject must name the colour the word is written in. |
| | |
| **Self-generation tests** | |
| Interval production | Subjects must generate intervals, typically by tapping a finger or foot or by saying something, typically once a second. The actual regularity is measured. |
| Random generating | Subjects must produce letters, digits, days of the week as randomly as possible. |
| | |
| **Memory** | |
| Digit span | A set of digits is presented to the user one digit at a time. Immediately afterwards, the subject must recall the digits. If correct, the sequence of digits gets longer and longer until they fail to recall all the digits. |
| Item recall | Lists of digits, letters, nonsense syllables and words are presented and the subject must recall them. |
| Memory search | Sets of target symbol sets are presented each followed by a probe symbol. Subject must say whether the probe symbol is a member of the target set. |

(continued)

| Memory | |
|---|---|
| Shopping list | A list of items is presented. The subject is then given a box containing the items on the list together with an equivalent number of items not on the list. The subject must pick out the items on the list. |
| QRST test | The letters Q, R, S and T are presented randomly. The subjects must count the occurrence of each letter and report the counts when asked. |
| Face recognition | A set of photographs is presented to the subject who must recognize them from a larger set. |
| Incidental memory | Subjects are not given specific information to remember but are asked to recall incidental features of the test or situation. |

Information on circadian rhythm can also be established from cognitive testing. A number of neurobehavioral and bodily functions including cognitive performance, core body temperature and certain hormones all vary as a function of circadian rhythm. In particular, short-term memory, cognitive performance and alertness all vary with respect to an individual's circadian rhythm. Tests such as addition tasks, digit symbol subtraction tasks, probe recall tasks and psychomotor vigilance tasks have all been shown to vary with circadian rhythm. These variations are also closely coupled to the individual's core body temperature. (Reference: 'Principles and practice of sleep medicine, 4th edition', Meir H. Kryger, Thomas Roth, William C. Dement, Elsevier Saunders ISBN 0-7216-0797-7.)

Whilst it is generally agreed that most cognitive tasks cannot be learnt, i.e. you can not learn faster reaction times, there can be a practice effect associated with such tasks that result in rapid improvements over a short period of time. To accurately determine information on circadian rhythm from cognitive performance, users should be trained to asymptotic levels before assessment in order to take practice effects into account.

The consumption of drug compounds can also have an impact on sleep quality that may or may not be associated with a corresponding increase or decrease in cognitive performance. This includes prescription and illegal drugs. For example, caffeine is very good at increasing alertness for a short period of time, but can lead to more disrupted sleep.

Making improvements to general sleep hygiene is often recommended to people who suffer from poor sleep quality. This encourages behaviours that promote sleep and discourages behaviours that hinder sleep. There are certain aspects of lifestyle that can impact on quality of sleep, including diet, exercise and stress levels. Environmental factors such as temperature, light, noise, humidity and comfort can also impact on sleep quality. Sleep hygiene involves identifying which of these factors promote or hinder sleep for a particular individual and then establishing a routine which actively promotes good sleep quality.

Good sleep hygiene also involves managing expectations; sleep becomes shorter, lighter and more fragmented as we get older. In some instances, sleep problems are aggravated by the expectation that 8 hours of sleep a night is necessary for good health when in fact 7½ hours may be perfectly adequate for certain individuals.

A feeling of satisfaction with one's sleep combines physiological changes occurring in our bodies during sleep with cultural and personal beliefs about the role of sleep, attitudes to life, life events, personality and mood. The following factors may help to improve sleep patterns:

- Maintain a regular sleep/wake schedule.
- Have a relaxing bedtime routine e.g. hot bath followed by reading.
- Avoid caffeine, alcohol, and nicotine close to bedtime.
- Exercise regularly (but not just before bedtime).
- Finish eating ca 2-3 hours before bedtime.
- Use your bedroom for sleep only.
- Create a sleep conducive environment that is dark, quiet, comfortable and cool.
- Sleep on comfortable mattress and pillows.
- Practicing relaxation techniques.

It is particularly important to follow a regular sleep/wake schedule, although a large majority of people often have sleep/wake schedules that are different whilst working and whilst off work, e.g. at the weekend. Having a dramatically different sleep/wake routine on days off often results in individuals feeling more tired when they return to work.

Regular sleep/wake schedules are particularly important for children whilst shift workers and people who travel across multiple time zones may find that their irregular routine negatively influences the quality of sleep they experience.

A number of behavioural strategies currently exist for improving sleep quality. The most common behavioural strategies are those for insomniacs who may be recommended to undergo sleep restriction or cognitive behavioural therapy for insomniacs (CBTi). Typically this involves restricting the amount of time spent in bed to the number of hours the patient thinks they sleep for each night (but never less than 5 hours) in order to ensure that the patient does fall asleep when they go to bed. This helps to re-establish a positive association between sleep and the bedroom. The patient is not allowed to nap or sleep whilst undergoing conditioning. Gradually, the amount of time the user can spend in bed is increased, provided their sleep efficiency scores remain above 80%, until a normal routine is established. Further to this, if the patient does not fall asleep within 15 minutes of going to bed, they have to get out of bed and not return to the bedroom until they are tired enough to sleep.

**Prior Art References**

The included reference listing provides additional background of interest in relation to the invention of which the documents discussed below are considered worthy of special note:

US 6,468,234 B1 (22 October 2002), "Sleepsmart", describes determining a sleep quality metric from a plurality of sensors embedded in a sheet. The sensors can monitor the subject's position, temperature and movement amongst other properties. The data from the sensors is correlated with lifestyle data which is gathered through the use of a questionnaire. The questionnaire returns subjective feedback on, for example, how well the subject perceives they slept, when they last ate a meal, estimate of stress levels and consumption of alcohol and caffeine. No objective measures of alertness are recorded whilst the user is awake.

US 2005/0042589 A1(24 February 2005), "Sleep quality data collection and evaluation", describes a method for collecting sleep quality data wherein sleep quality data collection is performed at least in part implantably. Both physiological and non-physiological parameters associated with sleep quality are measured. The patient can input information on the perceived quality of sleep, tobacco use and other self-described conditions. No objective measures of alertness are recorded whilst the user is awake.

WO 2005/066868 A2 (21 July 2005), "Sleep and environment control method and system", describes collecting objective environmental data whilst the user is asleep and correlates that with subjective data gathered when the user is awake and modifies the sleep environment depending on the results. No objective measures of alertness are recorded whilst the user is awake.

WO 2008/096307 A1 (14 August 2008), "Sleep management system", describes gathering objective data whilst the user is asleep and correlates that with subjective data when the user is awake in order to make recommendations to improve sleep. No objective measures of sleep quality are recorded whilst the user is awake.

US 6,743,167 B2 (1 June 2004), "Method and system for predicting human cognitive performance using data from an actigraph", describes determining the cognitive ability of a human to perform a task in the future using actigraphy. Performance is predicted for an individual based on that individual's prior sleep/wake history, the time of day and amount of time spent on a task. The patent defines a cognitive performance index which is a pre-defined function (i.e. baseline is set before any information about the user is established). It suggests that a user aims to alter their sleep/wake patterns in order to enable them to be at their optimal ability to perform a specific task (in the future) but does not describe how this may be accomplished in practice; the patent does not aim to improve a user's quality of sleep or quality of life. Variations in daytime performance can be different for different people, therefore predictive models do not apply equally well to different individuals; the predictive effect of changes in routine, medication, amount of exercise and changes in diet on cognitive performance are not easily determinable and are not accounted for here.

EP 1 618 913 A1 (25 January 2006), "Device for insomnia assessment and automated sleep behaviour modification", describes a device for automated sleep behaviour modification covering well known aspects of sleep hygiene and behavioural therapies. The device is preferably attached to the subject's wrist and the subject responds to prompts from which the computer determines whether the user is asleep or awake. The device does not monitor environmental or physiological parameters whilst the user is asleep and does not measure subjective or objective parameters whilst the user is awake.

US 7 366 572 B2 (29 April 2008), "Controlling therapy based on sleep quality", describes an implantable medical device which determines values for one or more metrics that indicate the quality of a patient's sleep, and controls delivery of a therapy based on the sleep quality metric values. This patent does not measure objective parameters whilst the user is awake.

US 2008/0157956 A1 (3 July 2008), "Method for the monitoring of sleep using an electronic device", describes a method where sleep sensor signals are obtained via a mobile communication device from sensor devices. In one embodiment, the mobile phone receives data from sleep sensors (e.g. a pressure sensor) via a short range radio connection. One embodiment uses questions to assess how the user felt they slept and when they started to feel tired, whether they ate a lot, engaged in sports and other general lifestyle data. Furthermore, various household appliances may also be automatically operated as a function of the sleep data on the mobile phone, e.g. putting the coffee machine on. Objective measures of sleep are not measured whilst the user is awake.

US 2006/0224047 A1 (5 October 2006), "Sleepiness prediction apparatus and sleepiness prediction method", describes a sleepiness prediction apparatus based on sleep history and subjective feedback from the user. It does not monitor environmental parameters whilst the user is asleep and does not measure objective parameters whilst the user is awake. The device is worn on the wrist with a sensor attached to a finger.

US Patent 5,479,939 (2 January 1996), " Sleep detecting apparatus" describes methods of detecting movement of a person in bed without contacting the body employing an infra-red sensor or sensor comprising a piezoelectric element disposed on the bedding, e.g. fixed on the surface of the mattress, such that it is deformed by body movement. However, there is no discussion of correlating data from such sensors for a person with objective test data on cognitive and /or psychomotor performance for the same person in waking hours with a view to assessing sleep quality and thereby formulating recommendations for improving this.

There remains a need for a consumer system that can provide a reliable description of an individual's sleep/ wake routine. In order to get an accurate description of one's sleep, endogenous and exogenous factors which may affect circadian and homeostatic drivers must be taken into consideration; none of the prior art addresses all of these factors in sufficient detail. The current invention represents a system that can correlate cognitive performance with environmental, physiological and lifestyle influences in a non-invasive format.

**SUMMARY OF THE INVENTION**

Establishing sleep quality involves more than determining a user's sleep architecture and asking them how they felt they slept; it is about correlating both how lifestyle affects how one sleeps and how that correlates to performance when awake. Sleep quality is affected by changes in the way in which we live. Whilst scientific experiments have been carried out by professionals in controlled environments to establish a fundamental understanding of sleep and the factors that influence it, there remains a need for a personalised system that can correlate an individual's sleep quality and behaviour with an objective measure of how they perform during the day. The present invention addresses this problem by providing in one aspect a method of managing the sleep of a user, the method comprising: (i) monitoring, using at least one sensor, one or more objective parameters relevant to sleep quality of the user when in bed, said parameters being selected from physiological parameters and environmental parameters and including at least movement of the user and /or electrical signals indicative of brain activity of the user; (ii)collecting, using a sensor unit, signal data from said at least one sensor and communicating the signal data to a processing means; (iii) collecting, using a portable user interaction device, objective test data from the user when awake, the objective test data being indicative of cognitive and/or psychomotor performance and communicating the objective test data to said processing means; and (iv) processing data from said sensor unit and said user interaction device to generate at least a combined sleep indicator metric, which takes both sensor unit data and cognitive and/or psychomotor data into consideration, or a cognitive and/ or psychomotor performance metric together with a sleep metric or a sleep quality metric; and (v) receiving at the portable user interaction device information relating to the user's sleep behaviour for display to the user.

The invention provides in another aspect a sleep management system for managing the sleep of a user, the system comprising: (i) at least one sensor for monitoring one or more objective parameters relevant to sleep quality of the user when in bed, said parameters being selected from physiological parameters and environmental parameters and including at least movement and/or electrical signals indicative of brain activity of the user; (ii) a sensor unit which collects signal data from said at least one sensor and communicates data to a processing means; (iii) a portable user interaction device, e.g. a mobile phone, which can collect objective test data, e.g. periodically or sporadically, from the user when awake indicative of cognitive and/or psychomotor performance, optionally together with other data relevant to assessing sleep quality, which may include, e.g. periodically or sporadically, subjective feedback from the user on sleep-related parameters, and which additionally communicates data to said processing means and receives information from the processing means which is displayed to the user, and (iv) said processing means, which may be present in either of said sensor unit or portable user interaction device or separate therefrom, and which processes data from said sensor unit and said portable device whereby at least a combined sleep indicator metric, which takes both sensor unit data and cognitive and/or psychomotor data into consideration, or a cognitive and/ or psychomotor performance metric together with a sleep metric or a sleep quality metric can be displayed to the user via the portable user interaction device.

Another aspect of the invention provides a method of managing the sleep of a user, the method comprising: receiving, at a processing means, signal data from at least one sensor, the data being indicative of one or more objective parameters relevant to sleep quality of the user when in bed, said parameters being selected from physiological parameters and environmental parameters and including at least movement of the user and /or electrical signals indicative of brain activity of the user; receiving, at the processing means, objective test data from the user when awake, the objective test data being indicative of cognitive and/or psychomotor performance; and at the processing means generating at least a combined sleep indicator metric, which takes both sensor unit data and cognitive and/or psychomotor data into consideration, or a cognitive and/ or psychomotor performance metric together with at least one sleep metric or sleep quality metric; and sending information relating to the user's sleep behaviour to a portable user interaction device for display to the user.

The invention will be described in more detail below with reference to the Figures.

**BRIEF DESCRIPTION OF THE FIGURES**

Figure 1 is a block schematic diagram of one embodiment of a sensor unit suitable for use in the invention.

Figure 2 is a block schematic diagram of one embodiment of a portable user interaction device suitable for use in the invention.

Figure 3 is a block schematic diagram of one embodiment of a processing unit (16) suitable for use in the invention.

Figure 4 details communications between the sensor unit, portable unit and processing unit.

Figure 5 details how the system can communicate with other external devices, e.g. a laptop.

Figure 6 teaches one embodiment of collecting data from an ambient noise sensor.

Figure 7 teaches one embodiment of collecting data from a movement sensor.

Figure 8 illustrates one manner of user input.

Figure 9 illustrates a second manner of user input.

Figure 10 illustrates a third manner of user input.

Figure 11(a) and 11(b) illustrates methods to determine baseline cognitive performance.

Figure 12(a), 12(b) and 12(c) illustrates methods to account for practice effects.

Figure 13 teaches one embodiment of mode I of the system.

Figure 14 teaches one embodiment of mode II of the system.

Figure 15 teaches one embodiment of mode III of the system.

Figure 16 teaches one embodiment of mode IV of the system.

Figure 17 teaches one embodiment of mode V of the system.

Figure 18 teaches one embodiment where behavioural changes are implemented.

Figure 19 teaches one relationship between reaction times and time of day.

Figure 20 teaches one relationship between reaction times and perceived alertness.

Figure 21 shows movement data of a user recorded using a piezoelectric cable and ambient light data collected via an ambient light sensor.

Figure 22 is a block flow diagram illustrating principal steps of a method according to one embodiment of the invention.

## DETAILED DESCRIPTION OF PREFERRED EMBODIMENTS

Provision of a plurality of sensors capable of monitoring different parameters that can have an effect on, or are indicative of, sleep quality will generally be favoured. The sensor unit will preferably collect data from a movement sensor and also sensors for parameters such as temperature, noise, humidity and light. The portable user interaction device will record objective measures of sleep, related to cognitive performance, and therefore circadian rhythm, and optionally may also record subjective measures such as perceived alertness and lifestyle data.

From the objective sensor data, and objective and, optionally, subjective data collected during the day, the system either determines at least one metric that contain information about the user's sleep and cognitive and/ or psychomotor performance or determines two or more metrics that, taken together, contain information about the user's sleep and cognitive and/ or psychomotor performance. The system may generate at least a combined sleep indicator metric, which takes both data from the sensor unit and cognitive and/or psychomotor data into consideration, or it may generate a cognitive and/ or psychomotor performance metric together with a separate sleep metric or sleep quality metric. The system may display the combined sleep indicator metric, or the cognitive and/or psychomotor performance metric and the separate sleep quality metric to the user, via the portable user interaction device. The user can decide how to use the information provided by the metric(s) in order to condition their behaviour in order to improve their quality of sleep. The user can preferably also access further information that may help them to improve their quality of sleep, for example the portable user interaction device may display further information including, as an example, recommended behavioural programs and/or actions for the user that, if followed, should lead to an improvement in the user's sleep quality. Furthermore, the user can decide to utilise the portable device to assist in behavioural conditioning in order to improve their quality of sleep and quality of life.

An objective measure of cognitive and/or psychomotor performance is useful because it can provide information on an individual's circadian rhythm; this information can be used by the user to optimise their lifestyle and sleep patterns. Objective measures of performance are also useful as they can help quantify improvements in sleep quality; a cognitive and/or psychomotor performance metric can be established that allows the user to compare their current cognitive and/or psychomotor performance level to previous cognitive and/or psychomotor performance levels.

The system may comprise a plurality of sensors, which communicate with a sensor unit or are integral to that unit, a portable user interaction device and said processing means which may be provided external to said sensor unit or portable device as an external processing unit; it may take the form of a software service accessible via a wide-area network, preferably the Internet.

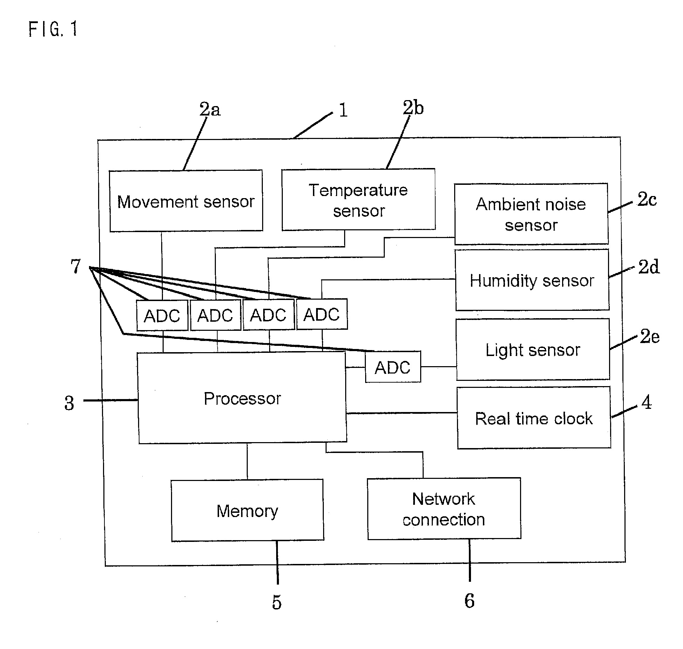

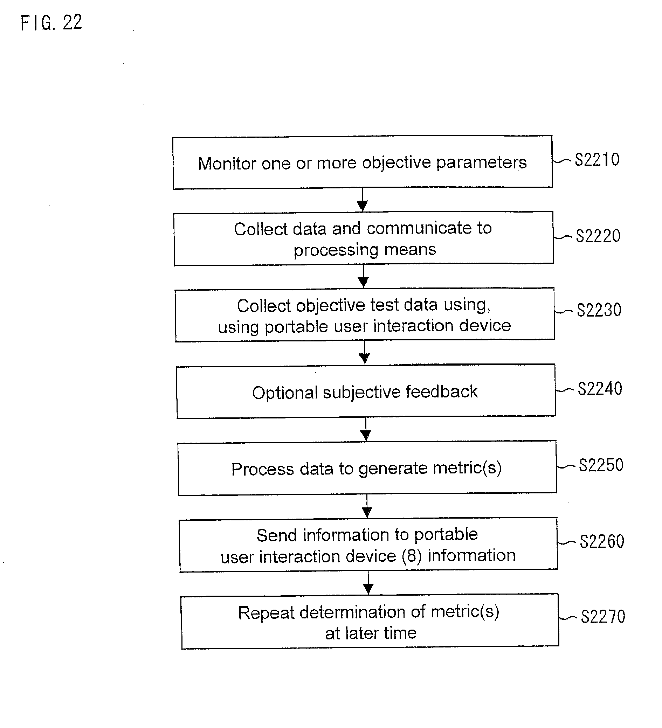

Figure 22 is a block flow diagram illustrating principal steps of a method of managing the sleep of a user according to one embodiment of the invention. Initially, the method comprises monitoring, using at least one sensor (for example one or more of sensors (2a-2e) described below with reference to Figure 1), one or more objective parameters relevant to sleep quality of the user when in bed (S2210). The parameter(s) is/are selected from physiological parameters and environmental parameters and including at least movement of the user and /or electrical signals indicative of brain activity of the user.

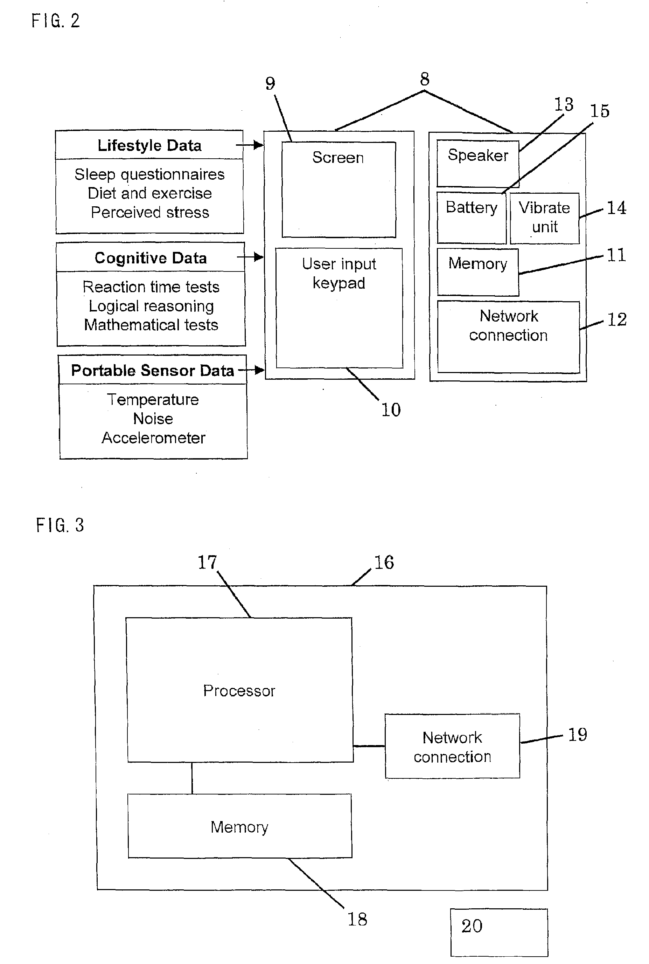

Next, the method comprises collecting, using a sensor unit (for example the sensor unit (1) described below with reference to Figure 1) signal data from said at least one sensor and communicating the signal data to a processing means (S2220) (for example the processing means (16) described below with reference to Figure 3).

Next, the method comprises collecting, using a portable user interaction device (S2230) (for example the portable user interaction device described below with reference to Figure 2), objective test data from the user when awake, the objective test data being indicative of cognitive and/or psychomotor performance and communicating the objective test data to said processing means.

Optionally, the method may further comprise supplementing said objective test data with subjective feedback from the user on sleep-related parameters (S2240), which may be input via said user interaction device.

Next, the method comprises processing data from said sensor unit and said user interaction device to generate at least a combined sleep indicator metric (S2250), which takes both sensor unit data and cognitive and/or psychomotor data into consideration, or a cognitive and/ or psychomotor performance metric together with a sleep metric or sleep quality metric.

Next, the method comprises receiving at the portable user interaction device information relating to the user's sleep behaviour for display to the user (S2260). The information relating to the user's sleep behaviour that is sent from the processing means to the portable user interaction device for display to the user may as one example comprise the metric(s) that were generated. Alternatively, the information may comprise information derived from the metrics, but not the metric(s) themselves. As a further example, the information may additionally or alternatively comprise some or all of the original data collected by the sensor unit and/or input to the portable user interaction device (S2270).

Preferably the method comprises repeating the generation of the combined sleep indicator metric at a subsequent time, or repeating the generation of the cognitive and/ or psychomotor performance metric together with a sleep metric or a sleep quality metric for the subsequent time. By generating the metric(s) at one or more subsequent times, the user is able to see, from a comparison of the sleep metric(s) obtained originally and the sleep metric(s) obtained at the subsequent time, whether any behavioural changes they have made have lead to an improvement in the user's sleep quality.

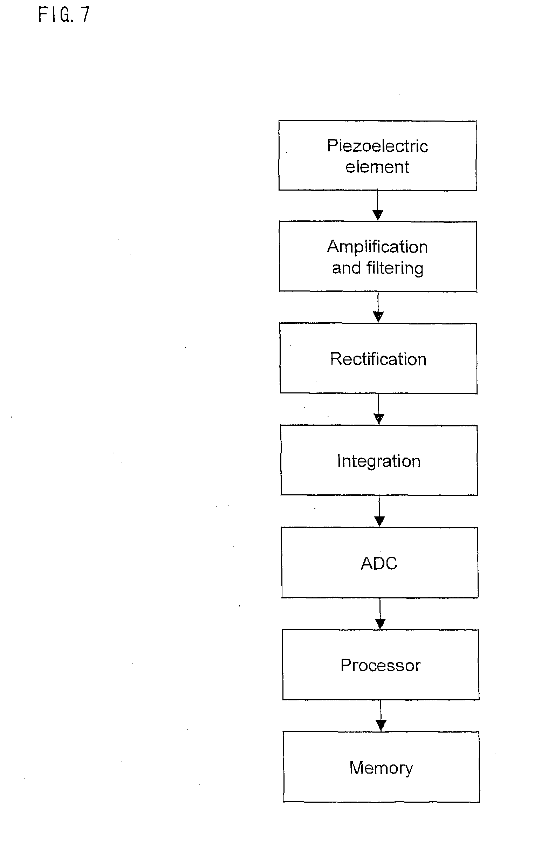

The sensor unit (1) desirably comprises at least one sensor, and more preferably a plurality of sensors (2a-e), a means for recording input from the sensors (4), hardware and/or software to carry out data processing as may be necessary and a network connection (6) so as to send data to and receive data from the processing means which also receives data from the portable user interaction device. Figure 1 shows one embodiment of a sensor unit (1) suitable for use in the invention. The sensor unit (1) of Figure 1 collects data from one or more sensors, in this example from a plurality of sensors including a movement sensor, a temperature sensor, an ambient noise sensor, a humidity sensor and a light sensor (2a-2e) and provides data from each sensor to a processor (3) via a plurality of analogue-to-digital converters (ADCs) (7). Processed sensor data is stored in a memory (5) with a time code provided by the real time clock (4) and sent to an external processing means, e.g. a processing unit as shown in Figure 3, via the network connection (6). The sensors are shown as integral to the sensor unit but one or more may be external to the sensor unit and connected to the sensor unit via a cable or wireless connection. For example, the movement sensor may comprise a piezoelectric element on the bed of the user and connected to the sensor unit by a cable whereby the sensor signal is processed as shown in Figure 7.

The processor (3) in the sensor unit repeatedly samples the sensors, preferably at a low frequency (of the order of 1 Hz, for example at a frequency in the range from 0.1 Hz to 100Hz). As noted above, the sensor unit will desirably contain a real-time clock (4) which provides a time code for each sensor data reading. In this case, each sensor reading may be stored in the memory (5) along with the time code. A time code may be stored for every sensor reading or for every multiple of sensor readings, for example every 100 readings. The sensor data stored in the memory is sent, for example periodically or on request, to the processing means via the network connection (6). It may be desirable to do further processing on the data before sending it to the processing means since the amount of data recorded may be large. The internal sensor processor (3) may therefore compress the data before sending, or may analyse the data to extract sleep metrics or sleep quality metrics and send the sleep metrics or sleep quality metrics to the processing means instead of sending the full data set. The sensor unit may be powered by a direct mains connection or battery. Other implementations of the sensor unit are also possible.

The portable user interaction unit (8) comprises a means of communicating with the user (9), a means of inputting information from the user (10) and recording input from the user (11), hardware and/or software to carry out any data processing as may be necessary and a network connection (12) so as to send data to and receive data from the processing means which also communicates with the sensor. Figure 2 shows one embodiment of a portable user interaction device (8) suitable for use in the invention. The portable user interaction device (8) of Figure 2 has a display screen (9), user input keypad (10) memory (11) and network connection (12) for sending data to and receiving information from an external processing means. The portable unit allows the user to interact with the system via the display (9) and an input device, such as a keyboard (10), mouse, touch screen, gesture camera, microphone or similar device. The portable unit communicates with the processing means to retrieve information to present to the user and sends data on user inputs using the network connection (12). The portable unit of Figure 2 also contains a speaker (13) and a vibration unit (14) which can be used to alert or prompt the user. A battery (15) or similar power system powers the portable unit so it may be carried easily without requiring a wire to a power socket. Other implementations of the portable user interaction unit are also possible.

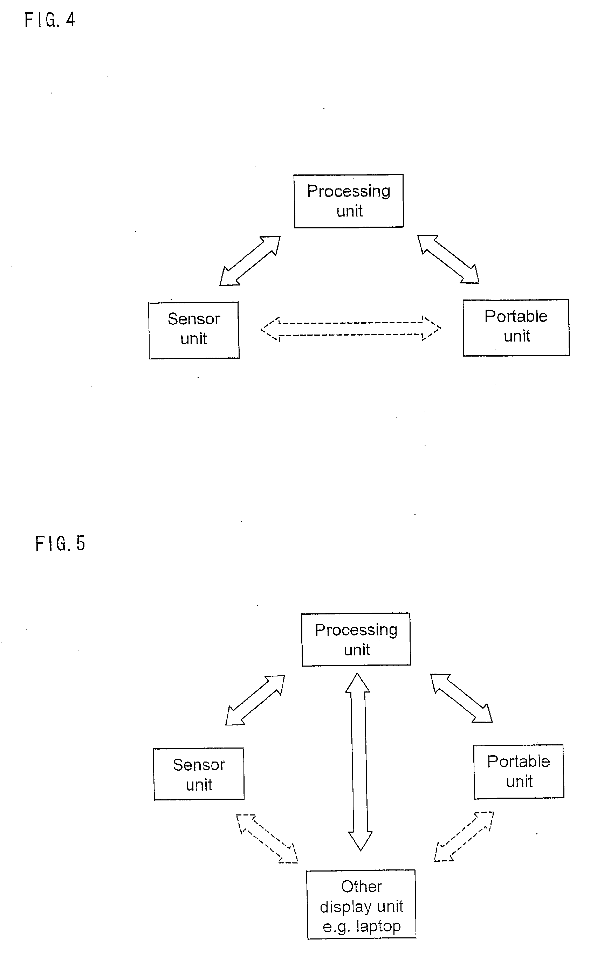

The processing means may comprise hardware and/or software to analyse data from the sensor unit and portable unit. It may be a processing unit disposed in either the sensor unit or the portable unit or may be a separate unit (see Figure 3). In any case the processing unit (16) contains a processor (17), memory (18) and network connection (19) such as to communicate with the sensor unit and portable unit (as indicated in Figure 4). Figure 3 shows one embodiment of a processing unit (16) suitable for use in the invention, for correlating data from the sensor unit and the portable user interaction device. The processing unit (16) of Figure 3 comprises a processor (17), memory (18) and network connection (19).

The program for operating the system and for performing any of the methods described hereinbefore may be stored in a program memory (not shown in Figure 3), which may be embodied as a semi-conductor memory, for instance of the well-known ROM type. However, the program may be stored in any other suitable computer-readable medium, such as magnetic data carrier, such as a "floppy disk", CD-ROM or DVD-ROM.

If the processing unit is disposed within an external unit, it may take the form of a software service connected to a wide-area network such as the internet. If the processing unit takes the form of a software service which communicates with the portable user interaction device and sensor unit via a wide-area network such as the internet, the processing unit software may be deployed on a single server computer, server cluster or as a cloud computer service. In this case a single processing unit may store and process the data from a plurality of handheld and sensor units used to monitor a plurality of users. Use of the processing unit may require the payment of a subscription or fee. The processing unit may also be able to communicate with other display devices, such as the user's desktop or laptop computer (Figure 5).

The sensor unit and portable unit may optionally be able to communicate directly with these other display devices. Furthermore, additional services may be offered to the user including having a sleep specialist examine the sensor data on behalf of the user.

The three components of the system, the sensor unit, the processing unit, and portable unit all contain network connections so as to be able to communicate with each other. If the processing unit is disposed within either the sensor unit or the portable unit it is possible that both units will share the same network connection.

Network technologies including WiFi (802.11), GSM, GPRS and 3G may be used to connect the system components to a wide-area network such as the Internet. Wired network technologies, which may be appropriate for the sensor unit or for an external server hosting the processing unit as a software service, include Ethernet. Other point-to-point network technologies including Bluetooth, ZigBee and Wireless USB may also be used to network the sensor unit, portable unit and processing unit together. The option also exists for the user to set the system up with no internet access.

The network connecting the sensor unit, processing unit and portable unit is preferably wireless so that the portable unit can be easily carried by the user without the necessity of a wired connection. A wired network connection may be possible for the sensor unit or processing unit.

The sensor unit desirably collects data from a plurality of sensors to monitor physiological parameters of the user whilst in bed and environmental parameters of the bedroom. These may include movement and one or more, preferably all, of temperature, light, and ambient noise sensors. The movement sensor(s) may be sufficiently sensitive to record the breathing and heartbeat of the subject. Thus, preferably a movement sensor may be employed which comprises a piezoelectric element disposed below the user in bed, for example as described in US Patent no. 5,479,939. Other sensors, such as humidity and pressure sensors, may be included if desired. The sensors are preferably non-invasive and non-obtrusive such that they do not disturb the sleep of the user. This data is collected and sent to the processing means for correlation with data from the sensor unit.

The sensors are in general implemented with a sensing element (2a-2e) and an analogue-to-digital converter (ADC) (7) to create a digital signal that can be analysed by a processor (3). Amplification and filtering of the sensor signal may be initially carried out.

A number of different methods may be employed for detecting the movement of an individual. These include using a sensor comprising a piezoelectric element as discussed above. Such a sensor may be a piezoelectric element comprising sheet, cable or film placed under the mattress or on the surface of the mattress of a sleeping individual as described for example in US Patent no. 5,479, 939, a capacitive sheet, cable or film placed under or on the mattress of a sleeping individual, infrared (IR) motion detector(s), for example as also described in US Patent no. 5,479,939, radiofrequency (RF) motion detector(s), or camera(s) to detect body movements. Actigraphy may also be used but is less preferred due to its obtrusive nature. Preferably a piezoelectric sheet, cable or film is deployed, desirably with the additional elements shown in Figure 7 present in the sensor unit providing amplification and filtering of the AC voltage signal followed by rectification and integration prior to digital conversion by an ADC as further discussed below.

As an alternative to or in addition to monitoring movement, monitoring of electrical signals indicative of brain activity, e.g. via an EEG headband or polysomnography, is possible with the current system provided that sleep related information can be determined from the data. However, these techniques are less preferred due to their obtrusive nature.

The temperature sensor may be implemented with a thermistor, thermocouple or similar device. The light sensor may be implemented with a photodiode or photoresistor and suitable amplification, preferably logarithmic.

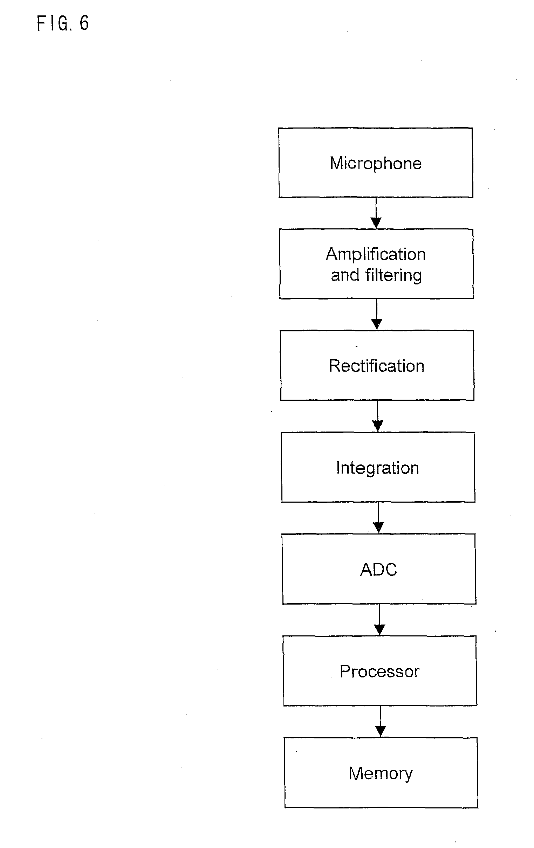

Figure 6 shows an implementation of an ambient noise sensor that can detect environmental noise or other noises whilst the user is asleep. Recording the ambient noise for an entire night at the typical sampling rate and precision of a CD or similar audio recording device requires a large amount of storage memory and a large amount of CPU time to process said data. Therefore the ambient noise sensor will preferably be designed to produce a slowly-varying signal that is characteristic of the loudness of the ambient noise that has occurred in the last few seconds. This signal can then be sampled at a much lower frequency (approximately 1 Hz) than the unprocessed audio signal and therefore requires very much less storage in the sensor unit and processing capability in the processing unit.

A compact microphone detects ambient noise and produces a small AC voltage signal. This is then amplified and filtered by circuitry which passes audio frequencies only (typically between 100 Hz and 20 kHz). The amplified AC signal is rectified by different circuitry. The rectified AC signal is then integrated. The integrator has a time constant of several seconds. In this way the AC signal representing a sound of duration less than a second becomes a DC signal that persists for several seconds. This DC signal is converted into a digital signal by the ADC. The processor (3) in the sensor unit then records the output of the ADC into a memory (5). Because the DC signal persists for several seconds, the processor can sample at a low frequency of the order of 1 Hz and will not miss any sounds than occur between sampling periods. Other implementations of an ambient noise sensor could be used in the system.

Figure 7 additionally shows an implementation of the system required to process the electrical signal from a piezoelectric film or cable in order to produce a slowly-varying signal that can be sampled at a low frequency (of the order of 1 Hz, preferably between 0.1 Hz and 100 Hz) by the sensor unit processor (3). For the same reasons as for the ambient noise sensor, sampling at a slow rate produces an advantageously small amount of data to be stored in the memory (5) of the sensor unit.

Referring to Figure 7, a piezoelectric sheet, film or cable produces a small electrical charge when it is compressed due to a movement of the user's body. The user's body may move due to a gross physical movement (such as moving an arm or leg) or smaller movements due to the process of breathing or the beating of the heart. The movement data will preferably be used to determine parameters such as, but not limited to, time to bed, time in bed, time out of bed, sleep onset latency, sleep architecture, number of night time awakenings, total sleep time, wake after sleep onset, sleep efficiency, heart rate and breathing. The small electrical charge caused by a movement appears as a small AC voltage signal across the capacitance of the piezoelectric element, e.g. cable. This AC voltage signal is then amplified and filtered by circuitry which passes low frequencies characteristic of movement, breathing and heart rate only (typically above 0.1 Hz and below 3 Hz). The amplified AC signal is then rectified. The rectified AC signal is then integrated. The integrator has a time constant of several seconds. In this way the AC signal representing a short movement becomes a DC signal that persists for several seconds. This DC signal is changed into a digital signal by the ADC (7). The processor (3) then records the output of the ADC into a memory (5).

The processing means can analyse the sensor data in order to calculate one or more sleep metrics. A "sleep metric" is a standard measurement used to describe sleep and includes information on one or more factors such as total sleep time (TST), time in bed (TIB), sleep onset latency (SOL), total wake after sleep onset (WASO), and number of awakenings after sleep onset (NWAK). Primarily, the movement data is analysed to calculate one or more sleep metrics, but other sensor data, especially light and noise, may also be used along with the movement data in order to calculate sleep metrics.

Additionally or alternatively, the processing means may analyse the sensor data in order to calculate one or more sleep quality metrics. A "sleep quality metric" is a measurement which can be used to describe the quality of sleep of a user such as sleep efficiency (SE), significant movement (SM) or sleep fragmentation (SF). A sleep quality metric may measure one of these, or may be a metric based on a combination of two or more of these.

Sleep efficiency is defined as the proportion of time in bed that is spent sleeping and can be calculated as follows:

$$SE = \text{Total time in bed spent sleeping/ Total time in bed}$$
$$= TST/TIB$$

Total sleep time (TST) can be calculated using the following formula:

$$TST = TIB - SOL - WASO$$

Sleep efficiency (SE) may be presented to the user as a percentage, with higher percentages indicating better quality sleep.

A significant movement (SM) sleep quality metric is a sleep quality metric based on the number of significant movements the user has whilst asleep. This is achieved by analyzing the movement data overnight, setting a threshold value and counting the number of movements above this threshold. This data can be presented to the user as a total number of significant movements during the night or as a number of significant movements per hour during the night.

A sleep fragmentation (SF) sleep quality metric monitors how fragmented a night's sleep the user had. This is based on the number of awakenings after sleep onset (NWAK) and may include the duration of these awakenings. In one embodiment, this sleep quality metric may only count awakenings that are longer than a specified duration, e.g. 5 minutes. This sleep quality metric can be presented to the user as a total number of awakenings during the night, or an average number of awakenings per hour.

Alternatively, a sleep quality metric may combine one or more these metrics in order to provide a combined sleep quality metric. In the general case, this combined sleep quality metric is calculated as a function of a number of the metrics given above and others:

$$\text{Sleep quality metric} = f(SE, SM, SF, ...)$$

For example, in one embodiment the combined sleep metric could be calculated as the sum of the sleep efficiency (SE), scaled to a number from 0 to 100, significant movement (SM), scaled to a number from 0 to 100, and sleep fragmentation (SF), scaled to a number from 0 to 100.

$$\text{Sleep quality metric} = s(SE) + s(SM) + s(SF)$$

where s() represents the scaling function. This yields a sleep quality metric from 0 to 300, indicative of the quality of the user's sleep.

Further sleep quality metrics may also take into account sensor data, behavioural data and lifestyle data. This may be in addition to the sleep quality metrics described above and may include factors such as bedroom temperature, irregular 'to bed' and 'rise' times, and amount of caffeine and / or alcohol consumed.

Other sleep quality metrics may be implemented with the current method and system.

The portable user interaction device enables cognitive and/or psychomotor performance to be measured whilst the user is awake. It also preferably allows the user to input subjective information on their lifestyle and quality of sleep at times convenient to them. The portable user interaction device may be implemented as a custom piece of hardware with dedicated software or the functionality of the portable user interaction device may be implemented as software on a general-purpose device. The portable user interaction device may be a dedicated handheld device, a mobile phone, a wrist watch, a blackberry, a PDA, laptop computer, an e-book, an alarm clock, or other device, including the extra functionality of those devices. Furthermore, the portable unit may be docked with the sensor unit to charge or exchange data. The user interaction device may also incorporate a plurality of sensors selected from, for example, movement, temperature, light and humidity sensors. These may be used to monitor exogenous factors whilst the user is awake. The portable user interaction system can also provide an alerting function via a built-in speaker, vibrating unit, messaging service or other alerting methods. The software can also make use of the network connection of the general purpose device.

In some embodiments, the combination of hardware units for implementing a sleep management system can comprise the required sensor(s), the sensor unit and the portable user interaction device(s) which can collect objective test data periodically or sporadically from the user when awake indicative of cognitive and/or psychomotor performance.

In some embodiments, in the combination of hardware units the or each portable user interaction device can present to the user one or more tests selected from mathematical processing, logical reasoning, spatial processing, reaction time, tracking, attention/ vigilance, self-generation cognitive function tests, memory tests.

In some embodiments, in the combination of hardware units the or each portable user interaction device can passively monitor an activity of the user when awake indicative of cognitive and/ or psychomotor performance.

In some embodiments, in the combination of hardware units the or each portable user interaction device provides a keyboard or keypad whereby speed and /or accuracy of typing can be monitored.

In some embodiments, in the combination of hardware units the or each portable user interaction device is a mobile phone.

A cognitive and / or psychomotor performance metric is a measurement used to describe how well a person has performed on a cognitive and / or psychomotor test. The measure of the cognitive and / or psychomotor performance metric depends on the test undertaken. For example, the metric for reaction time tests is the reaction time of a single test or the average reaction time over several tests. For tests that involve mathematical processing, spatial processing, attention / vigilance or memory, the metric is the percentage of correct answers or percentage of correct answers within a given time. Other cognitive and / or psychomotor performance metrics may exist for different cognitive or psychomotor tests. The cognitive and / or psychomotor metric displayed to the user will be appropriate to the test undertaken. As used herein, the term "performance metric" is taken to mean a metric of cognitive and / or psychomotor performance.

A sleep indicator metric preferably combines one or more sleep metrics or sleep quality metrics with one or more cognitive and / or psychomotor performance metrics. In the general case, such a "combined sleep indicator metric" is calculated as a function of at least one sleep metric and/or sleep quality metric and at least one cognitive and / or psychomotor performance metrics:

$$\text{Sleep indicator metric} = f(\text{sleep metric(s), sleep quality metric(s), cognitive and / or performance metric(s)})$$

For example, in one embodiment a combined sleep indicator metric could be calculated as the sum of the sleep efficiency (SE), scaled to a number from 0 to 100, and a psychomotor metric representing a response time (PMM), also scaled to a number from 0 to 100:

$$\text{Combined sleep indicator metric} = s(SE) + s(PMM)$$

where s() represents the scaling function. This yields a combined sleep indicator metric from 0 to 200, indicative of the quality of the user's sleep and psychomotor performance.

Other combined sleep indicator metrics may be used with the current invention.

Changes in sleep quality and / or performance will be reflected in at least one of the sleep or sleep quality and / or cognitive and / or psychomotor performance metrics or the combined sleep indicator metric. In one example, factors that improve the user's sleep quality should result in an improvement to at least one of the metric scores whilst factors that cause poorer quality sleep should result in reduction of at least one of the metric scores. The processing unit will be able to automatically calculate and track changes in the sleep quality and performance metrics and the user can also track any changes in these metrics manually.

Cognitive and / or psychomotor performance of the user may be measured through presentation to the user via said portable user interaction device of one or more tests selected from mathematical processing, logical reasoning, spatial processing, reaction time, tracking, attention/ vigilance, self-generation cognitive function tests and memory tests, but the processing means may also receive data indicative of cognitive and / or psychomotor performance derived from passive monitoring of an activity of the user when awake, e.g. information on speed and / or accuracy of typing at a keyboard or keypad. This may be via the portable user interaction device or another source as exemplified by the discussion in embodiment 11 below.

In the initial set-up phase, the user can input personal details such as age, sex, height, weight, existing medical conditions, what goals they want to achieve, what time they have to start and finish work, what days they work, whether work is regular or irregular, how they currently perceive their sleep, typical to bed and rise times, medication, prior sleep problems and any other relevant information, preferably via the portable user interaction device.

If the user presents an existing medical condition, or appears to be at high risk from a recognized sleep disorder, then a recommendation to see a health professional will be made.

The sensor unit is set up in the user's bedroom. When used for the first time, the user may have to place a movement sensor, such as the preferred piezoelectric sheet, on the bed or beneath the sheets upon which they sleep. The movement sensor may be held in place using elastic, Velcro, clips or other attachment methods and is connected to the sensor unit via a cable or wireless connection.

The user is then preferably guided through a series of steps to ensure that the system is set up correctly. This involves checking that the sensor unit (1) and portable unit (8) can record information from their various inputs. Once it is established that the sensor unit and portable unit can record information from the various sensors and other inputs, the user checks that the sensor unit and portable unit can communicate with the processing means (16). Once the system is set up correctly, the user is allowed to proceed with using the system. If an error is returned during the set-up phase, then the user is presented with an error message, preferably via the portable user interaction device, along with steps on how to overcome the problem.

The system is now ready for use. The general cycle of the system is to use the sensor unit to record objective data at least whilst the user is asleep or is trying to go to sleep, and to record objective and subjective data using the portable unit whilst the user is awake. The processing means (16), indicated above as preferably a software service provided via the Internet, processes all the information from the sensor unit and portable unit and establishes correlations between the data. The results, including at least the sleep metric(s) and/or sleep quality metric(s), cognitive and / or psychomotor performance metric(s) or the combined sleep indicator metric(s), are presented back to the user, along with behavioural recommendations if required, and the cycle continues. Preferred implementation of this process is described in more detail below.

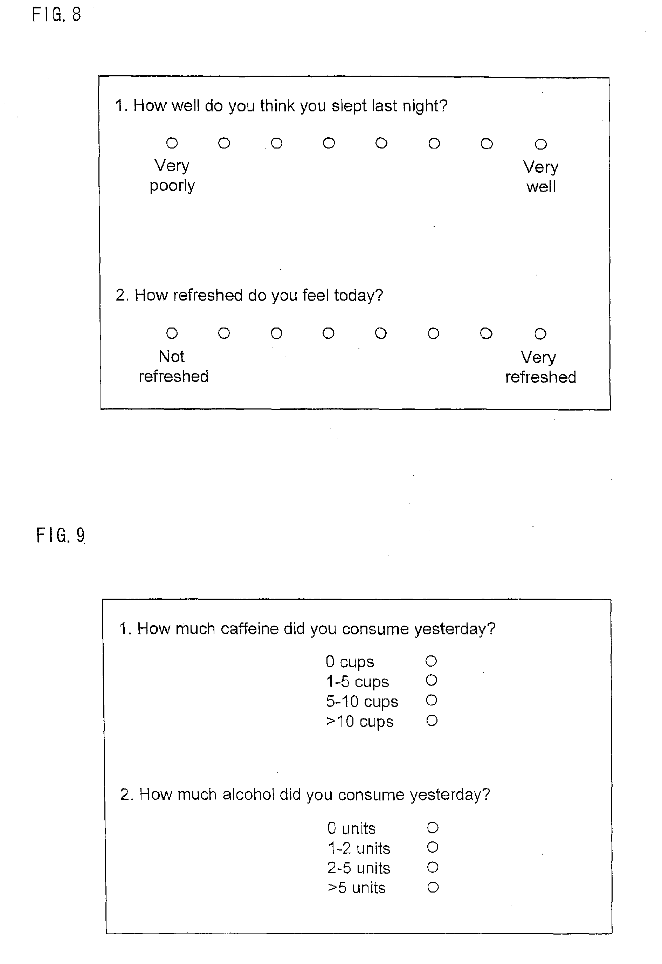

When the user is awake, they answer some subjective questions on their quality of sleep using the portable user interaction device. This information may be collected through the use of standard questionnaires such as the EPS and/or SSS, and by filling out an electronic sleep diary which may include questions such as "How do you think you slept?", "How do you feel this morning?", "Did you consume any alcohol?", "Did you feel ill or unwell?", "How much exercise did you do" and "Did anything unusual happen today?" (Figure 8). The user can answer such questions when they wake up, before they go to bed or during the day when they have some spare time. In this instance, the portable user interaction device acts as an interactive electronic sleep diary. If the user fails to use the portable unit during the day the extra information can be added later or omitted entirely. This data is collected and sent to a processing unit (16).

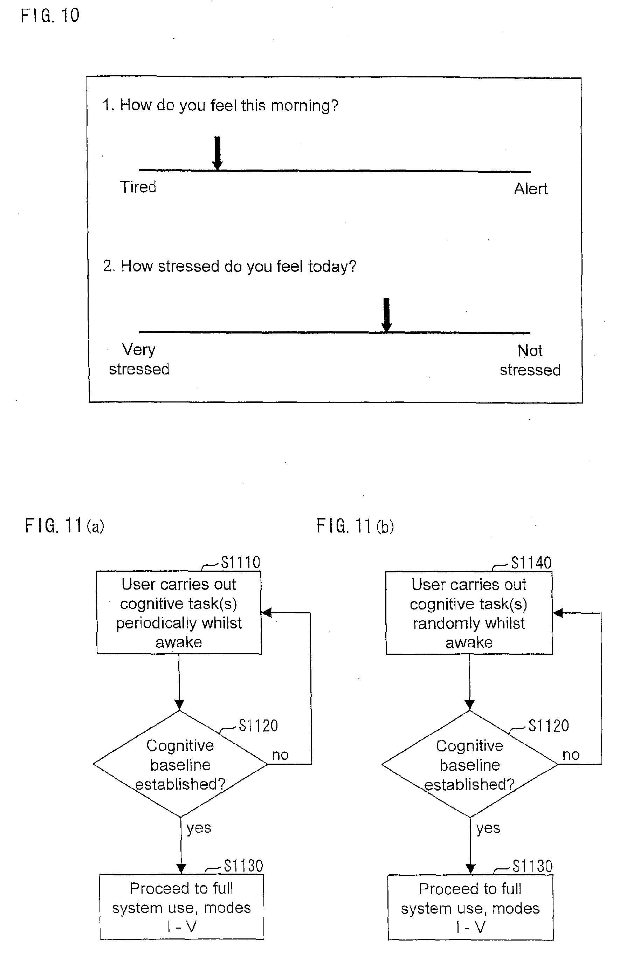

The input to the portable user interaction device may be through, when a question is displayed on the display of the portable user interaction device, selecting one of a series of options from a drop-down menu displayed on the display of the portable user interaction device, through highlighting a radio button on a suitable scale displayed on the display of the portable user interaction device (Figure 9), by placing a marker on a sliding scale (Figure 10) displayed on the display of the portable user interaction device or typing a response. Other methods of data input are also acceptable.

As already noted above, it is well known that individuals are not always able to accurately determine their level of performance or sleepiness. Subjective measures of sleepiness and performance can all be influenced by factors such as mood, time of day, perceived poor sleep ("I think I slept poorly therefore I must be tired"). Hence, an objective measure of daytime performance will provide much more accurate information on how well an individual has slept and may help to break false assumptions a user has with their quality of sleep.

Objective measures of cognitive and / or psychomotor performance can be measured whilst the user is awake using the portable user interaction device. This provides information on the user's cognitive and / or psychomotor performance, from which information on the user's circadian rhythm can be inferred. As circadian rhythm, and therefore cognitive and / or psychomotor performance, varies over the course of a day, baseline curves must be established.

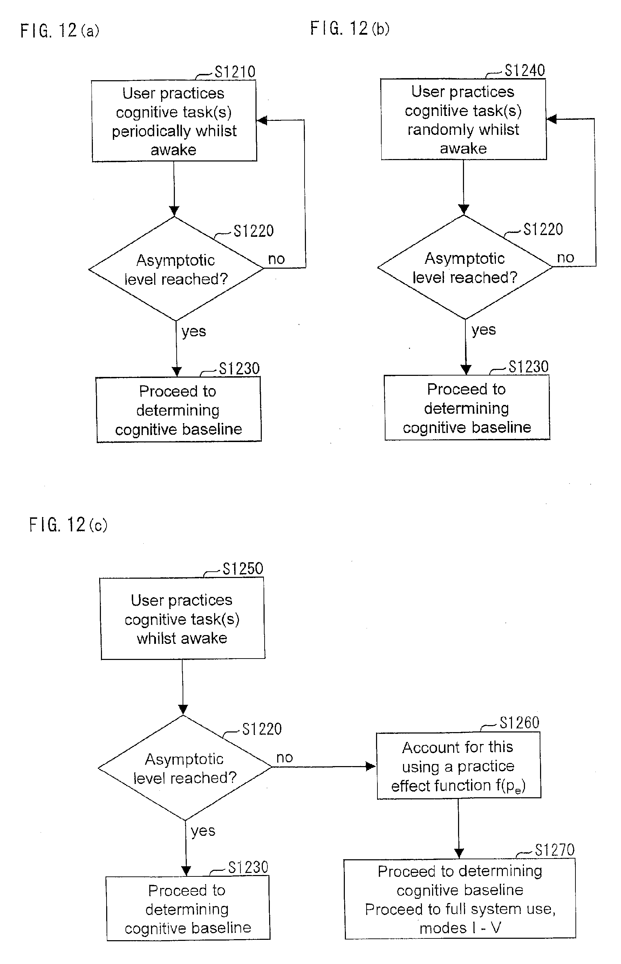

A baseline for cognitive and / or psychomotor performance, and therefore circadian rhythm, can be established in a number of ways all involving the user carrying out multiple cognitive and / or psychomotor tests whilst awake, for example as shown in Figures 11(a) and (b) and 12(a)-(c). In the method of Figure 11(a) a user carries out cognitive and / or psychomotor task(s) periodically whilst awake (S1110), and it is then determined whether a cognitive and / or psychomotor baseline has been established (S1120). If no baseline has been established, the user carries out further cognitive task(s) until it is determined that a cognitive and / or psychomotor baseline has been established. The method then proceeds to full system use (S1130), for example according to one of Modes I to IV as described below. The method of Figure 11(b) is similar to the method of Figure 11(a), except that the user carries out cognitive and / or psychomotor task(s) randomly or sporadically whilst awake (S1140), rather than periodically. Ideally, cognitive and / or psychomotor tests are carried out several times a day, over a wide range of times, for several days. Preferably tests are carried out between every 1 to 8 hours whilst the user is awake for a period of days, preferably from 1 day to a few weeks. In one embodiment, the user may be prompted by the portable device to carry out cognitive and / or psychomotor tasks at the same times every day for several days. These times may be desirably pre-defined by the processing unit (16) in order to gain the maximum amount of information on cognitive and / or psychomotor performance and circadian rhythm using the least number of tests so as not to be a burden on the user. Alternatively, the portable device may prompt the user to carry out a cognitive and / or psychomotor task at several random times throughout the day for several days. The times would be determined such that tests were carried out over as wide a range of times as possible (without affecting when the user went to bed or got up). Another embodiment would involve the user carrying out multiple cognitive and / or psychomotor tests, at times convenient to them, for several days. Users may be encouraged to carry out a minimum number of cognitive and / or psychomotor tasks every day to ensure that enough information has been recorded in order to establish baselines for cognitive and / or psychomotor performance and therefore circadian rhythm. Testing at different times of day can also help to identify true variations in circadian rhythm. The baseline period ends when sufficient data has been recorded in order to establish a performance curve, and/or circadian rhythm curve, with a variance below a particular threshold. This may take a few days. If it is not possible to measure a cognitive and / or psychomotor baseline, it is acceptable to use a pre-defined function for cognitive and / or psychomotor performance that may be updated as the system is used; however, this may not be as accurate as a personalised cognitive and / or psychomotor performance curve. It is also a feature of the present invention to allow the cognitive and / or psychomotor performance curve to be updated, particularly when the user implements behavioural changes.

In order to establish an accurate cognitive and / or psychomotor baseline, practice effects must also be taken into consideration. A number of methods exist to take practice effects into account. One method involves the user practising cognitive and / or psychomotor tasks until asymptotic levels are reached. This could involve the user performing cognitive and / or psychomotor tasks at set times throughout the day. Preferably, this is carried out before establishing a cognitive and / or psychomotor performance baseline. , For example in the method shown in Figure 12(a), a user practices cognitive and/or psychomotor task(s) periodically whilst awake (S1210), and it is then determined whether an asymptotic level has been reached (S1220). If an asymptotic level has not been reached, the user carries out further cognitive and/or psychomotor task(s) until it is determined that an asymptotic level has been reached. The method then proceeds to determining a cognitive and / or psychomotor baseline (S1230). Alternatively, the user may perform cognitive and / or psychomotor tasks randomly or sporadically throughout the day (S1240) as in the method of Figure 12(b) - which is similar to the method of Figure 12(a), except that the user carries out cognitive and / or psychomotor task(s) randomly or sporadically whilst awake, rather than periodically. An alternative method would be to take a pre-defined function, a practice effect function $f(p_e)$, detailing how people usually improve when performing cognitive and / or psychomotor tasks and use an algorithm to take the practice effect into account and adjust the cognitive and / or psychomotor results accordingly, as in the method of Figure 12(c). For example in the method shown in Figure 12(c), a user practices cognitive and / or psychomotor task(s) whilst awake (S1250) (this may be periodically, randomly or sporadically), and it is then determined whether an asymptotic level has been reached (S1220). If it is determined that an asymptotic level has been reached, the method then proceeds to determining a cognitive and / or psychomotor baseline (S1230). However, if an asymptotic level has not been reached, a practice effect function $f(p_e)$ that takes account of the fact that the user's performance of the cognitive and / or psychomotor task(s) has not yet reached an asymptotic level is determined (S1260), and the method then proceeds to determining a cognitive and / or psychomotor baseline (S1270). This allows practice effects to be taken into consideration and allows the user to begin using the system immediately. This method could be made more accurate by counting the number of times an individual has carried out a specific cognitive and / or psychomotor task and using software to take that into account and adjusting the results accordingly.

Once a cognitive and / or psychomotor baseline is established, cognitive and / or psychomotor measurements can be reduced in frequency to as little as once a day. The baseline performance curve can be used to take into account time of day effects on cognitive and / or psychomotor tasks enabling cognitive and / or psychomotor tasks carried out at different times to be compared accurately. Furthermore, any change in circadian amplitude or circadian phase may also be monitored via cognitive and / or psychomotor performance and compared to the circadian rhythm baseline.

Performance and circadian rhythm are also closely correlated with core body temperature, so optionally a body thermometer may be used with the current system in order to help determine baseline curves.

The processing unit (16) collects and processes all the subjective and objective information, relevant to sleep quality, gathered from questionnaires, diaries, sensors and measures of performance via the sensor unit (1) and portable unit (8). This information can then be analysed, correlated and presented back to the user in an easy to read format. The processing unit (16) can help the user to identify which parameters result in poorer quality sleep, which parameters help to improve their quality of sleep or whether there was a trigger for a period of poor sleep. This is achieved by tracking the sleep metric(s) and/or sleep quality metric(s) and / or cognitive and / or psychomotor performance metric and / or the combined sleep indicator metric. The user can then condition their behaviour in order to improve their quality of sleep. The impact of any behavioural change can be tracked using the sleep metric(s) and/or sleep quality metric(s) and / or cognitive and / or psychomotor performance metric and / or the combined sleep indicator metric. The information may be presented back to the user via the portable user interaction device or, alternatively, on a computer, TV, phone, PDA or other display.

Records of the subject's sleep are built up over a period of time; days, weeks, months and years. The data is stored in a memory, preferably the memory (18) of the processing unit (16). The user may request sleep reports on certain aspects of their sleep when desired. Some reports will require only a few days worth of data, for example information on basic sleep/wake times, whilst other reports will require much more data, particularly reports detailing slowly varying parameters such as stress, medication or weight loss effects on sleep quality. Users also have the option to create their own reports from the data that is stored in the memory. The information may be presented back to the user via the portable user interaction device or on a computer, TV, phone, PDA or other display.

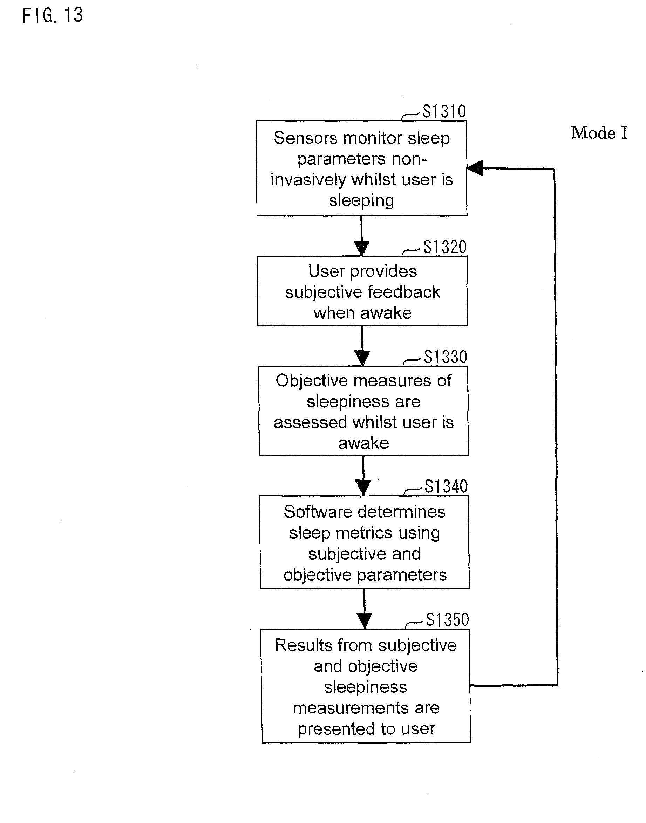

The system may be used in several different modes. Mode I is where the system simply records objective and subjective sleep related information (both when the user is awake and asleep) and presents the data back to the user (Figure 13). In the method of Figure 13, one or more sensors monitor sleep parameters non-invasively whilst the user is sleeping (S1310). The user provides subjective feedback when awake (S1320). One or more objective measures of sleepiness are assessed whilst user is awake (S1330), for example relating to cognitive and/or psychomotor performance. One or more sleep metrics and/or sleep quality metrics are then determined using the subjective and objective parameters (S1340), and finally results from subjective and objective sleepiness measurements are presented to the user (S1350).

The information recorded may include, but is not limited to, time to bed, time in bed, time out of bed, sleep onset latency, sleep architecture, number of night time awakenings, total sleep time, wake after sleep onset, sleep efficiency, amount of exercise, consumption of alcohol, consumption of caffeine, perceived alertness, perceived levels of stress and cognitive and/or psychomotor performance. Other parameters may also be measured. The data may be presented back to the user as raw information, pictorially, graphically, as a factual sleep report, as a combined sleep indicator metric, or as a series of sleep metrics which may include a sleep quality metric and a cognitive and/or psychomotor performance metric. Preferably, the combined sleep indicator metric will take at least data from the sensor unit and cognitive and/or psychomotor performance data into consideration. Other options for data presentation are possible.

The simplest implementation of Mode I would involve recording objective sleep data whilst the user was asleep, objective data relating to cognitive and/or psychomotor performance whilst the user was awake and displaying at least the combined sleep indicator metric, which takes both sensor unit data and cognitive and/or psychomotor data into consideration, or a cognitive and/or psychomotor performance metric together with a sleep metric or a sleep quality metric.

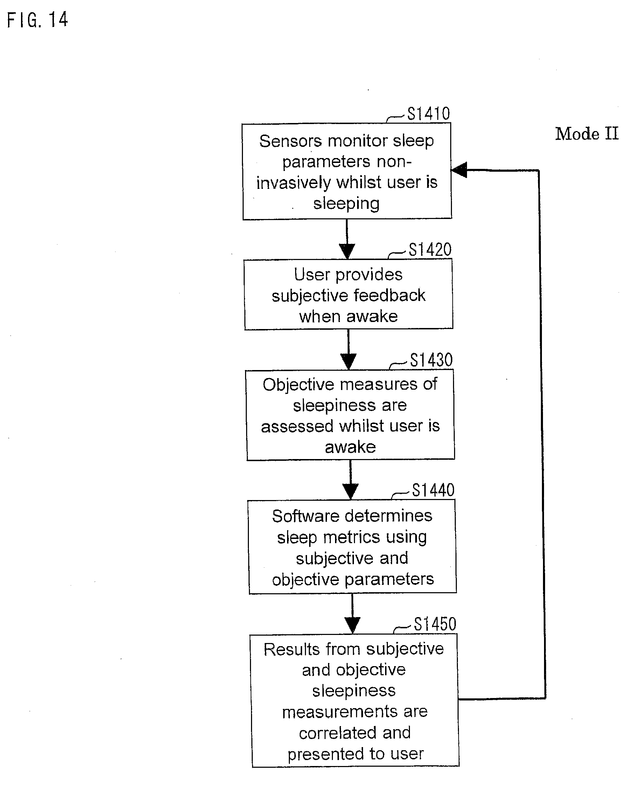

Mode II is where the user would like the processing unit (16) to identify any correlations between the recorded data and present that information back to the user, in addition to the information available in mode I. A method according to mode II is shown in Figure 14 and, as can be seen, in the method of Figure 14 one or more sensors monitor sleep parameters non-invasively whilst the user is sleeping (S1410). The user provides subjective feedback when awake (S1420). One or more objective measures of sleepiness are assessed whilst user is awake (S1430), for example relating to cognitive and/or psychomotor performance. One or more sleep metrics or sleep quality metrics are then determined using the using subjective and objective parameters (S1440). Finally, results from subjective and objective sleepiness measurements are correlated and are presented to the user (S1450). In this method, either or both positive and negative correlations may be identified. For example, the system may identify that the user sleeps poorly when caffeine is consumed in the evening (a "negative" correlation), or that the user sleeps better on days when they've done an hour's exercise (a "positive" correlation). As a further example, the processing unit may identify that the user's cognitive performance results are consistently better in the morning than in the afternoon.

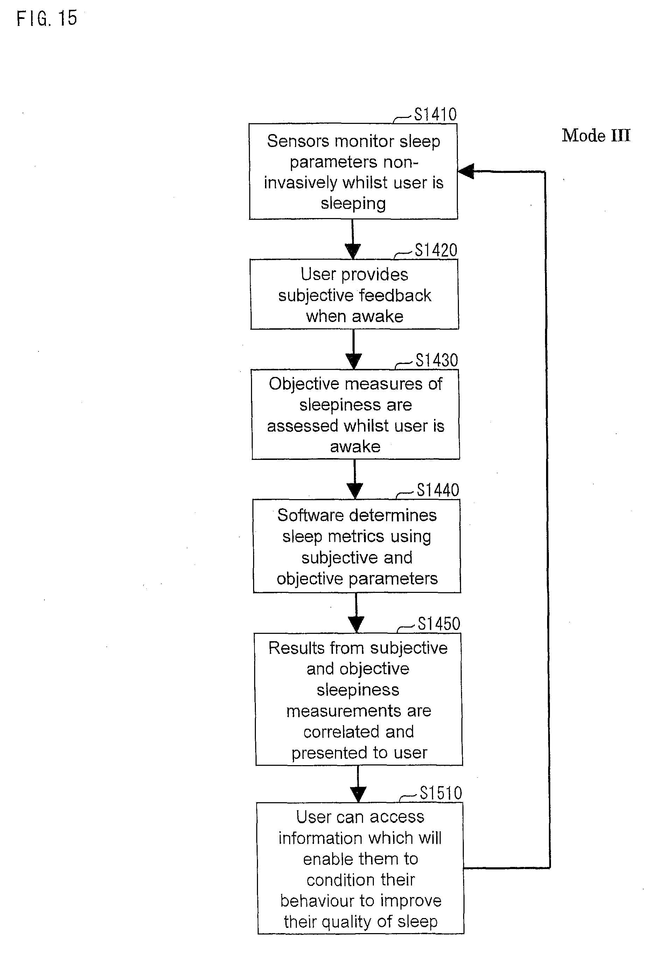

Mode III further presents information relevant to the correlations identified in mode II. One example of a method according to Mode III is shown in Figure 15. The first 5 features (S1410) - (S1450) of Figure 15 correspond to the features of Figure 14, but the method of Figure 15 further includes the feature of making it possible for the user to access information which will enable them to condition their behaviour to improve their quality of sleep (S1510). As one example, this information may include information on sleep hygiene. For example, if the sleep sensor system identifies that a user always suffers from poor sleep and never does any exercise, then it may draw attention to the user of the benefits of exercise in improving sleep quality, as described in general good sleep hygiene rules, and display a behavioural recommendation to do more exercise. Similar correlations between drinking too much alcohol and poor sleep quality may also be drawn to the attention of the user along with a behavioural recommendation to drink less alcohol. Or if the user displays an irregular sleep/wake schedule then the sleep hygiene rule that suggests that a regular sleep/wake schedule can help to improve sleep quality will be highlighted along with a behavioural recommendation to maintain a regular sleep routine.

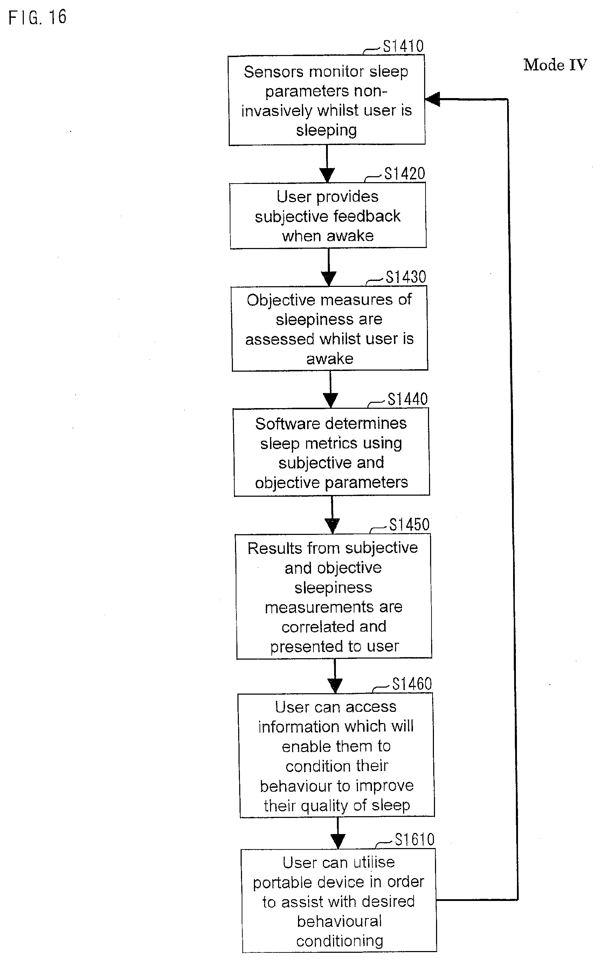

Mode IV enables the user to utilize the system in order to help them condition their behaviour in order to improve their sleep quality. One example of a method according to Mode IV is shown in Figure 16. The first 6 features of Figure 16 (S1410) - (S1510) correspond to the features of Figure 15, but the method of Figure 16 further includes the feature of the user utilising the portable user interaction device in order to assist with desired behavioural conditioning (S1610). As one example, any changes in behaviour can be registered by the user with the system utilising the portable user interaction device and any improvements in sleep quality tracked. For example, further information presented to the user may include customized times for going to bed and waking up, particularly if an irregular sleep schedule has been identified. Customised times for going to bed and waking up may be provided in response to user input or may be automatically calculated by the processing means using the sleep metrics, for example from the total sleep time (TST) and average times for going to bed and waking up, and presented to the user. If the user chooses to decline the automatically generated times, they are able to input their own times.

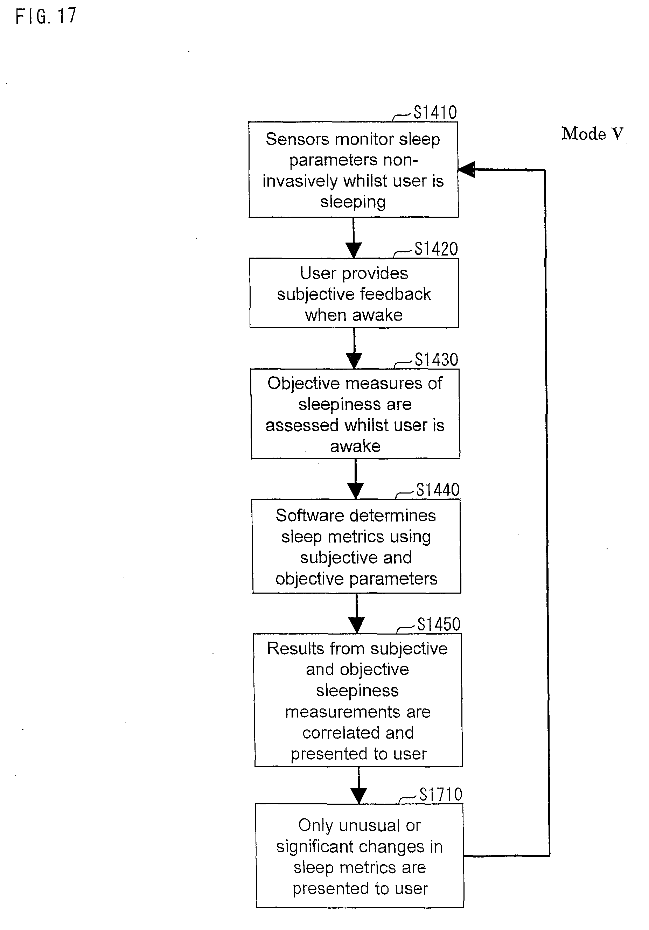

In mode V the system only informs the user of significant or unusual changes in their sleep behaviours. One example of a method according to Mode V is shown in Figure 17. The first 5 features (S1410) - (S1450) of Figure 17 correspond to the features of Figure 14, but the method of Figure 16 further includes the feature that only unusual or significant changes in sleep metrics are presented to the user (S1710).

In all modes of operation, the combined sleep indicator metric, which takes both sensor unit data and cognitive and/or psychomotor data into consideration, or a cognitive and/ or psychomotor performance metric together with a sleep metric or sleep quality metric which is displayed to the user can help the user track what effect any lifestyle or behavioural changes has on their quality of sleep and / or performance whilst awake. The processing unit can also track changes in these metrics.

It is preferable to have established the cognitive and/or psychomotor baseline before using modes I-V (this allows the cognitive and/or psychomotor performance metric to take time of day effects into consideration so that cognitive and/or psychomotor performance metrics can be accurately compared).

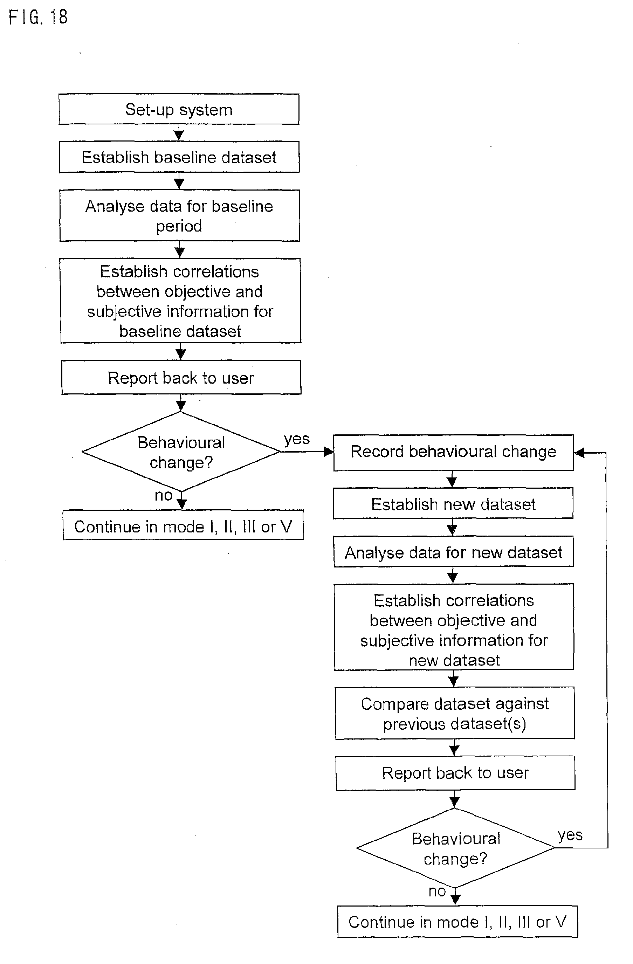

Furthermore, before using mode IV, a baseline dataset describing the user's current sleep/wake patterns, performance and lifestyle habits is preferably established. This dataset will be defined as the baseline dataset and involves recording objective and subjective data for a period of time until sufficient information is gathered to get an overview of the user's typical daytime and evening habits along with sleep and performance metrics. The time required to establish a baseline dataset may be several days to several weeks; baselines for users with regular sleep/wake routines will be established more quickly than those with irregular routines. The baseline dataset will be established either when the system identifies patterns in the dataset or when a pre-defined time period has elapsed, preferably between 1-8 weeks. Alternatively, if the user has already been using the system in modes I-III for a period of time then this information may be sufficient to form the baseline dataset, including baseline sleep quality and cognitive and / or psychomotor performance metrics, and / or sleep indicator metric. The baseline dataset provides the system with an overview of the user's current habits; future datasets can then refer back to the baseline dataset in order to make comparisons and to assess progress. If the user then decides to change their behaviour, they can register the behavioural change, such as deciding to go to bed and get up at the same time every day, and record a new dataset, defined as the 'new routine 1' dataset here, with the behavioural change implemented. The same parameters will be measured as were measured in the baseline, including updated sleep or sleep quality and cognitive and / or psychomotor performance metrics, and / or combined sleep indicator metric, to allow for comparisons to be made. The processing unit (16) can then compare the new dataset, 'new routine 1', with the baseline dataset in order to measure whether sleep quality and/or performance has improved by comparing the sleep or sleep quality and cognitive and / or psychomotor performance metrics, and / or sleep indicator metric. If necessary, the cognitive baseline can be updated to reflect the impact of the behavioural change. The system can also monitor compliance of implementing behavioural changes automatically via the sensors and/or via questioning. If sleep quality still has not improved, the user may decide to change another behaviour and go through the cycle again, forming a new routine 2 dataset. At the end of this third cycle, the processing unit can compare sleep or sleep quality and cognitive and / or psychomotor performance metrics, and / or combined sleep indicator metric with all the previous datasets it has stored in its storage unit. If and when the user decides not to change any behaviours, the system may be used in modes I, II, III or V (Figure 18).

In modes I-V, the system may also access a network or the internet in order to gain access to information that may be of relevance to the user. For example, if the sensor system finds a correlation between high levels of pollution and/or pollen with the user experiencing poorer quality sleep, then the system can alert the user to weather forecasts predicting high levels of pollution and/or pollen.

From the above it will evident that the following advantages are gained by implementing a sleep management system in accordance with the invention. Firstly, whilst there is prior art for general sleep systems that monitor sleep parameters whilst asleep, a system does not currently exist that takes into account both data recorded whilst the user is asleep and objective parameters affected by sleep recorded whilst the user is awake. Instead, the prior art relies on the user inputting subjective data at the start or end of the day in the form of a questionnaire. This subjective data can be unreliable. The present invention is an improvement over the prior art in that it enables objective and subjective measures of important sleep related parameters to be made non-invasively during the periods when the user is awake at a time that is convenient to said user. This gives a more accurate measure of cognitive and/or psychomotor performance at different points during the day and provides data from which information on circadian rhythm can be deduced. The current invention analyses and correlates the effects of endogenous and exogenous stimuli which may affect an individual's quality of sleep in order to give them a more accurate overview of their sleep/wake patterns.

Correlation between subjective measures on sleep quality, lifestyle factors, objective measures of sleep quality and performance are particularly important as the true factors influencing an individual's sleep are not necessarily the same as the factors that an individual believes affects their quality of sleep. The true factors that affect an individual's sleep quality and / or performance whilst awake will be reflected in the sleep or sleep quality and cognitive and / or psychomotor performance metric(s), and / or combined sleep indicator metric.

Actimeters can be used to monitor daytime movement or activity; however, this does not necessarily relate to an individual's cognitive performance or feeling of alertness; it is obvious that manual workers will generally be more active than an office worker for example but it doesn't necessarily translate that the manual worker therefore feels more alert than the office worker. The present invention has distinct advantages over actimetry in that it uses more accurate methods of recording information, i.e. by recording both objective and subjective measures of parameters relevant to sleep whilst the user is awake. The present invention is equally applicable to all occupations and can be used accurately, for example, by both manual and sedentary workers. Furthermore, actimeters have to be worn on an individual which can be uncomfortable. The present invention preferably enables monitoring of sleep related parameters non-invasively.

The current invention also provides advantages over carrying out cognitive and / or psychomotor tests in isolation of any other information; the current invention helps the user to understand their circadian rhythm and how they can positively influence their performance through making changes to their sleeping and lifestyle behaviours. Cognitive and / or psychomotor performance data can help provide the user with an objective measure of how well they have slept along with information on their circadian rhythm, allowing the user to optimise their lifestyle and sleep routine. Further to this, the cognitive and / or psychomotor performance metric may help the user break any false assumptions they have with regards to their quality of sleep. The current invention can further collect, detail and correlate information about an individual's circadian rhythm with cognitive and / or psychomotor performance, lifestyle, environmental and physiological parameters.

The preferred method in the prior art of monitoring circadian rhythm is to measure core body temperature; this frequently involves taking rectal temperatures. (High and low body temperatures correlate well with good and poor performance.) Alternatively, salivary melatonin levels may be measured. The current invention offers an advantage over the prior art in that it can help to provide information to a user on their circadian rhythm using non-invasive techniques.

Further to this, the user can interact with the system, preferably via the portable user interaction device, which can help the user guide their behaviour in order to improve their quality of sleep and therefore their general wellness and quality of life.

Furthermore, if the user decides to utilise the portable user interaction device in order to assist with any behavioural conditioning, then the portable user interaction device can be used as a source of support and information. Preferably, the processing means monitors efficacy of and /or compliance with any behavioural program such as CBTi (cognitive behavioural therapy for insomnia) or action presented to and chosen by the user and provides (i) one or more of warning, guidance, advice and message(s) of encouragement to the user to aid efficacy of and/or compliance with any chosen program or action and /or (ii) one or more updated recommendations for behavioural changes and / or actions. The system may permit setting of a customized behavioural program or action.

The current system is suitable for use over a wide range of ages, provided the user is capable of inputting objective test data to the portable user interaction device. The system may be adapted for use by an older child or adolescent. The portable user interaction device may, for example, present games or tasks specifically to encourage use by a child and at the same time collect data on cognitive and/ or psychomotor performance. For example, interaction with the portable device may be via an animated character which will be attractive to the child. The information presentation may be adapted to ease understanding by a child. Different or additional behavioural programs may be available for selection and presentation where the system is intended for child use.

A system may be provided with an additional identical portable user interaction device for use by a second user, in the case of two people sharing a bed as further discussed in the exemplification of implementation of the invention below.

The present invention can provide the user with an objective description of their sleep/wake patterns. This further presents advantages over the prior art in that the present invention does not rely solely on subjective information which can be inaccurate, nor does it focus on a single objective aspect of sleep information, i.e. sensors recording sleep parameters whilst the user is asleep. Accurate information on how an individual performs during the day allows the present invention to find more accurate correlations with other subjective and objective sleep parameters.

The following exemplification sets out specific applications of the invention by way of example only. In all embodiments, the impact of any lifestyle or behavioural change on sleep quality and / or performance will be reflected in the sleep or sleep quality metric(s) and performance metric(s), and / or the combined sleep indicator metric. Changes in these metrics can be tracked automatically by the processing unit and / or manually by the user.

**Examples**

**Embodiment 1**

A system that returns a daily sleep or sleep quality metric and a cognitive and/or psychomotor performance metric, or a combined sleep indicator metric, based on subjective and objective measures of a user's sleep parameters, after detailed monitoring over a period of time.

Each day sensors monitor objective physiological and environmental parameters (movement, temperature and light), whilst the user is asleep. The user enters subjective sleep data via questionnaires and / or an electronic sleep diary when awake using the portable user interaction device.

The user further uses the portable user interaction device to objectively measure their performance when awake via cognitive and / or psychomotor performance tests. Multiple measures of cognitive and / or psychomotor performance whilst the user is awake allow the circadian rhythm of the subject to be detailed.

The results of subjective and objective measures of sleep recorded whilst the user was awake and asleep are presented to the user for their information; the processing unit (16) calculates if the user is receiving a typical amount of sleep, sleeping at typical times, waking a typical number of times during the night and displaying other typical sleep behaviours. Details of how cognitive and / or psychomotor performance changed during the course of each day are presented to the user as are correlations between subjective and objective measures of user's sleep. This data is presented to the user via the portable device in a clear format. A cognitive and / or psychomotor performance metric based on the recorded information is also displayed for the user, along with a sleep metric or sleep quality metric. Alternatively, a combined sleep indicator metric may be displayed.

Information that may help the user to improve their quality of sleep may be made available via e.g. the portable user interaction device or a device with a network connection to the processing unit and may include, for example, information on general sleep hygiene rules. The user can then decide whether or not they want to condition their behaviour in order to improve their quality of sleep. The impact of any behavioural change can be assessed via the sleep/sleep quality and cognitive and / or psychomotor performance metrics or the combined sleep indicator metric

For example, data analysis carried out by the processing unit (16) may recognise that the user does not have a regular sleep schedule which results in a poor sleep or sleep quality metric score. The user interaction device may present the rules of sleep hygiene to the user highlighting the fact that a regular sleep schedule can help to improve an individual's quality of sleep. If the user decides to enforce any of the rules of sleep hygiene, the effect of the lifestyle changes on the quality of sleep of the user will be reflected in the sleep or sleep quality and / or cognitive and / or psychomotor performance metrics or the sleep indicator metric.

In another example, the system might identify a correlation between environmental data and performance. One case may be where the user wakes up at sunrise due to light flooding into their bedroom. Analysis of the user's circadian rhythm may suggest that they are an evening person and their performance would improve if they could sleep later than, for example, a 06:30 sunrise. The user can then take measures to install black out blinds or use an eye mask in order to prevent waking at sunrise and waking at a time more conducive to the rhythms of their natural body clock.

In other instances, the system may identify a correlation between lifestyle data and performance. One example would be where the system identifies that the user has a regular sleep/wake routine during Monday to Friday when they are at work, but that this routine changes dramatically at weekends when they are off work. The system may also identify that the user also drinks a lot more caffeine at the weekends compared to during the week, that their perceived alertness is worse at weekends than during the week and that their cognitive performance is also worse at weekends. These correlations may be used to inform the user that they could improve their sleep quality by maintaining a regular sleep/ wake routine regardless of whether they are working or not and that this new regular routine would also help to prevent the need for consuming large amounts of caffeine at weekends in a bid to feel more alert. The new routine should result in improved perceived alertness at weekends and better performance scores.

In a further example, the system may identify that a user, who is a sports enthusiast, has a circadian rhythm that suggests that the user's ideal waking time is 06:30. Therefore, it may be recommended that they do not exercise late in the evening, say after 19:00, as that may interfere with their quality of sleep.

A further example is one where a user would like to optimise the time of their afternoon siesta; they may want to determine at what times their circadian rhythm dips in the afternoon and take their siesta at that time.

In a further example, the system may show a correlation between lifestyle data, environmental data and performance. One case may be where the system records low environmental temperatures in the bedroom, an increase in the consumption of hot caffeinated drinks and more restless sleep. The user may then condition their behaviour to consume less caffeinated drinks and increase the temperature in their bedroom in order to achieve more restful sleep.

In a further example, the system may help to change one's perception of their sleep. An individual may report very fragmented sleep and waking up for long periods of time during the night. Their perceived alertness may also score poorly. However, the analysis the system carries out on recorded subjective and objective information may indicate that the user's movement during the night is considered to be normal and that their cognitive scores are also very good, suggesting that they are probably getting an adequate amount of sleep. This may help to ease any anxiety the user has about their sleep patterns.

In another example, the system may help to identify a trigger for poor sleep.

In a further example, the generation of the sleep metric(s) may be repeated at a subsequent time, to generate a combined sleep indicator metric for the subsequent time, or a cognitive and/ or psychomotor performance metric together with a sleep metric or sleep quality metric for the subsequent time. The metric(s) for the subsequent time may then be compared with the initial metric(s), and any changes in the metrics are indicative of changes in the user's sleep behaviour. This makes it possible to, for example, determine whether any changes that the user has made to their behaviour, or any actions they have undertaken, have improved the user's sleep quality. The process of generating the metric(s) at a subsequent time may be repeated, periodically or sporadically, for as long as it is desired to monitor the user's sleep quality.

**Embodiment 2**

A system as in embodiment 1 where the user decides that they would like assistance in order to condition their behaviour in order to improve their quality of sleep.

The portable user interaction device can be utilised in order to provide assistance in guiding the behaviour of the user, therefore helping them to comply with their desired sleep/wake schedule or improve their sleep hygiene.

For example, the user may decide to try and maintain the sleep schedule they have when working on their days off. The user can request that the user interaction device displays a message to this effect on the evening before their next day off. The alarm on the portable user interaction device would also automatically go off at the same time as it normally would when the user was working.

In another example, the user may decide to employ the portable user interaction device in order to help them establish a relaxing bedtime routine. An alarm or prompt may instruct the user to begin winding down and give them enough time to do a quiet and relaxing task such as taking a bath or reading a book prior to going to sleep.

In another example, the system may have established that the user spends long periods of time in bed without sleeping. Therefore, if the user spends longer than 15 minutes in bed without falling asleep, as detected by the sensor unit, then the portable user interaction device may instruct the user to get out of bed and not to return to bed until they feel tired enough to sleep.

The effect of any lifestyle and / or behavioural change on an individual's sleep quality and / or performance whilst awake will be reflected in the sleep or sleep quality and / or cognitive and / or psychomotor performance metrics or the combined sleep indicator metric.

**Embodiment 3**

A system as in embodiments 1-2 that can monitor the behaviours of two people who share a bed, i.e. couples.

The system comprises the same environmental and physiological sensors as the individual system, but is adapted to be suitable for two people. There are two portable user interaction devices such that each person can fill out details relevant to their sleep quality at times suitable to each individual. Preferably, the portable user interaction device of one user is identical to the portable user interaction device of the other user.

The software system is able to correlate information from all sensors and both user interaction devices to provide information on both users' quality of sleep.

The system can help to identify factors, such as one person moving excessively during the night or differences in circadian rhythms, which may affect the quality of sleep of the individual and/or their partner. Analysis may show a cross-correlation between improving a user's quality of sleep and an improvement in sleep quality of their bed partner. This may provide motivation for the user to maintain good sleeping habits when they realise what impact their sleep routine has on the sleep quality of their bed partner.

The effect of any lifestyle and / or behavioural change on an individual's sleep quality and / or performance whilst awake and their partner's sleep quality and / or performance will be reflected in their individual sleep or sleep quality and cognitive and / or psychomotor performance metrics, or their combined sleep indicator metric.

## Embodiment 4

A system as in embodiments 1-3 where the user experiences difficulty sleeping and requires assistance in resetting their sleep/wake schedule.

People who may want to reset their sleep/wake schedules include those who travel frequently (to help them to overcome jetlag), shift workers or those people who suffer from insomnia.

In one example the user may decide that they would like help resetting their sleep patterns. The user may be a poor sleeper and has been found to spend up to 10 hours in bed trying to sleep and only managing to sleep for 5 hours (giving a sleep efficiency of 50%). The system may inform the user of a program that may help them reset their sleep patterns, such as a cognitive behavioural program (for example, sleep restriction or stimulus control). For example, the system may recommend reducing the number of hours spent in bed to the average number of hours the user actually spends sleeping. In this example the user would reduce time spent in bed from 10 hours to 5 hours and ideally go to bed 5 hours before they wanted to get up. The portable user interaction device may be utilised in order to assist with the behavioural conditioning. For example, if the user is instructed to go to bed initially for 5 hours, then the portable user interaction device can prompt the user when to go to bed and when to get up via an alarm. Measures of performance can also be made periodically throughout the day which can help with monitoring the progress of the behavioural conditioning. Cognitive and/or psychomotor tasks may also provide a welcome distraction when the user is trying to stay up until the right time before going to bed and consequently can be a measure of compliance. The number of hours the user is permitted to spend in bed gradually increases to a target number (for example between 7-9 hours) providing their sleep efficiency remains sufficiently high (for example providing their sleep efficiency stays > 85%). This helps the user re-establish the association between sleep and the bedroom. Subjectively, the user may not initially feel as though their sleep quality is improving but objective results may show an improvement in sleep efficiency and/or performance over a period of days or weeks.

The effect of the behavioural change on an individual's sleep quality and / or performance whilst awake will be reflected in the sleep quality and cognitive and / or psychomotor performance metrics, or combined sleep indicator metric.

## Embodiment 5

A system as in embodiments 1-4 where the user can send data to a sleep expert for further analysis.

The sensor system may be used for a period of days or weeks in order to record objective and subjective information related to the users sleep quality. The user may decide that they would like an expert opinion on their sleep/ wake patterns and can send the data off to a medical professional for analysis. This may be achieved by sending the recorded data electronically via a secure internet connection or via traditional post.

The sleep expert can base their response by analysing the sleep metrics, sleep quality metrics and the cognitive and / or psychomotor performance metrics, or the combined sleep indicator metric.

## Embodiment 6

A system as in embodiments 1-5 whereby the user suspects that they, or a third party, may be suffering from a medical sleep problem. The recorded data can be used for a sleep consultation with a sleep expert. Following advice from a sleep professional, the system can be used to help implement the recommendations made. When the user returns for follow-up consultations, the data recorded from the sleep sensor system can be used by the medical professional to assess efficacy of treatment and to monitor compliance.

The medical professional can monitor the efficacy of any recommended behavioural changes on an individual's sleep quality and / or performance whilst awake by analysing the sleep metrics, sleep quality metrics and the cognitive and / or psychomotor performance metrics, or the combined sleep indicator metric.

### Embodiment 7

A system as in embodiments 5-6 whereby the sleep sensor system may be used to monitor compliance of a sleep behavioural program as specified by a sleep professional by collecting and presenting relevant information.

The medical professional can monitor the compliance of any recommended behavioural changes on an individual's sleep quality and / or performance whilst awake by analysing the sleep metrics, sleep quality metrics and the cognitive and / or psychomotor performance metrics, or the combined sleep indicator metric.

### Embodiment 8

A system as in embodiments 1-7 whereby the system helps to improve the quality of sleep in pregnant woman both pre and post childbirth.

Sleep patterns can be greatly disturbed in pregnancy, with typical changes including increased daytime sleepiness, increased fatigue, less slow wave sleep and more night time awakenings. Circadian rhythms and sleep architecture may also change during the course of pregnancy.

Users can track what behaviours and parameters result in an improvement in sleep quality or a decline in sleep quality over a period of time. Any changes in circadian rhythm can be monitored in detail using both subjective and objective parameters measured whilst the user is awake and asleep.

The effect of any lifestyle and / or behavioural change on an individual's sleep quality and / or performance whilst awake will be reflected in the sleep quality and / or performance metrics, or the combined sleep indicator metric.

### Embodiment 9

A system as in embodiments 1-7 whereby the sleep sensor system can help improve the quality of sleep in woman going through the menopause transition.

Sleep patterns can be greatly disturbed when going through the menopause, with typical changes including hot flashes, more night time awakenings and increased daytime sleepiness.

Users can track what behaviours and parameters result in an improvement in sleep quality or a decline in sleep quality over a period of time via the sleep or sleep quality and cognitive and / or psychomotor performance metrics, or the combined sleep indicator metric. Any changes in circadian rhythm can be monitored in detail using both subjective and objective parameters measured whilst the user is awake and asleep.

### Embodiment 10

A system as in embodiments 1-9 whereby the system can help to monitor the effects of going onto or coming off medication, e.g. antihistamines or sleeping pills, on general performance and sleep quality. The system can be further used to assess the effects of new medications in clinical trials.

It is well known that certain pharmaceutical compounds can have an impact on performance and sleep quality. However, in clinical trials it is difficult to distinguish between the placebo effect and true pharmacological effects, with drowsiness or sleepiness often listed as a common side effect. The current invention could be used to more objectively assess how new medications affect sleep quality and performance of volunteers in medical trials via the sleep or sleep quality and cognitive and / or psychomotor performance metrics, or the combined sleep indicator metric.

In another example, the system may be used by somebody who is taking sleeping pills. Sleeping pills are not designed for indefinite use, and users may find it useful to have an objective measure of their sleep/wake patterns and general performance whilst on and off medication in order to better manage their quality of sleep.

### Embodiment 11

A system as in embodiments 1-4 where in addition to using the portable user interaction device to measure cognitive performance when the user is awake, cognitive performance is also measured as the user carries out some other activity which has an additional purpose, so that data indicative of cognitive and / or psychomotor performance may be derived from passive monitoring of an activity of the user when awake, with the data being obtained from said portable user interaction device and/or from an additional source. For example, an office worker may spend large amount of time typing during the day. The speed of typing or accuracy of keystrokes may give a good indication of the office worker's level of cognitive performance. Information on the speed of typing or accuracy of keystrokes may be obtained from the user typing using a keypad on the portable user interaction device. However, by using hardware such as a camera, or software deployed on the office worker's computer, information on the speed of typing or accuracy of keystrokes may be obtained from the user typing using a keypad on the user's office computer, ie from a keypad provided separately from the portable user interaction device. That is, a cognitive performance metric can be obtained by monitoring the office worker's typing on their office computer, without requiring the user to type into the portable user interaction device..A metric obtained in this way has the advantage that the user does not need to dedicate time specifically to carrying out a cognitive performance test.

The additional cognitive performance metric obtained in this embodiment is used by the processing unit (16) along with the other objective and subjective measurements to calculate overall sleep metrics and find correlations as described.

In the above embodiments, the user provides subjective data such as subjective sleep data, and the information presented to the user may include some or all of the subjective data or data derived therefrom. The invention does not however require that the user provides subjective data and, in its most general form, requires only data relating to one or more objective parameters relevant to the user's sleep quality. Any one of the above embodiments may therefore be modified by eliminating the collection of subjective data from the user so as to use only data relating to one or more objective parameters relevant to the user's sleep quality.

The following examples illustrate data collection in accordance with the invention.

### Example 1

A user carried out a 3 minute psychomotor vigilance test, PVT, test four times at day for twelve days, with the tests performed at the same time each day. The tests were taken at 9am, 12pm, 4pm and 10:30pm and were chosen to fit in around the user's lifestyle. Before the 3 minute PVT test, the user was asked to estimate how alert they felt using the Stanford Sleepiness Scale.

| The Stanford Sleepiness Scale | |
|---|---|
| Scale rating | Degree of sleepiness |
| 1 | Feeling active, vital, alert or wide awake. |
| 2 | Functioning at high levels, but not at peak. Able to concentrate. |
| 3 | Awake, but relaxed. Responsive but not fully alert. |
| 4 | Somewhat foggy. |
| 5 | Losing interest in remaining awake. Slowed down. |
| 6 | Sleepy. Fighting sleep, prefer to be lying down. |
| 7 | Sleep onset soon. Having dream-like thoughts. |

The user was not required to cut stimulants, such as caffeine, out of their diet, nor were they required to maintain a strict daily routine of rising from and going to bed at the same time every day. They were also not required to carry out the PVT in an isolated environment free of any other distractions (which would not represent a real life scenario).

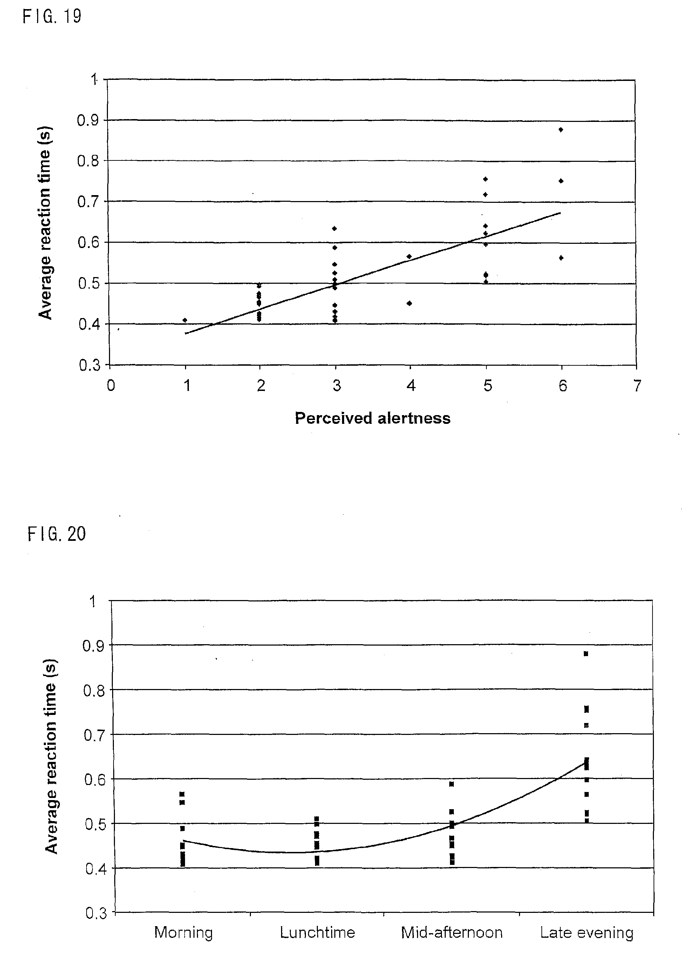

An average reaction time was recorded for each PVT test the user carried out. Figure 19 shows the average reaction time plotted against the time of day at which the test was carried out, while Figure 20 plots average reaction time against perceived alertness.

The data was analysed using a two-way ANOVA in order to establish any correlations between the time of day and the user's average reaction time. There was a significant correlation between reaction time and the time of day at which the test was performed; calculated F-ratio was 22.23 compared to $F_{3,33} = 4.44$ at 1 % significance level.

The data was also analysed in order to determine whether there was any correlation between the time of day and the user's perceived alertness. The F-ratio calculated was 49.35 which is greater than $F_{3,33} = 4.44$ at 1% significance level indicating a significant correlation between time of day and perceived alertness for this individual.

The data clearly shows that the user's reaction times generally increased late in the evening before going to bed.

### Example 2

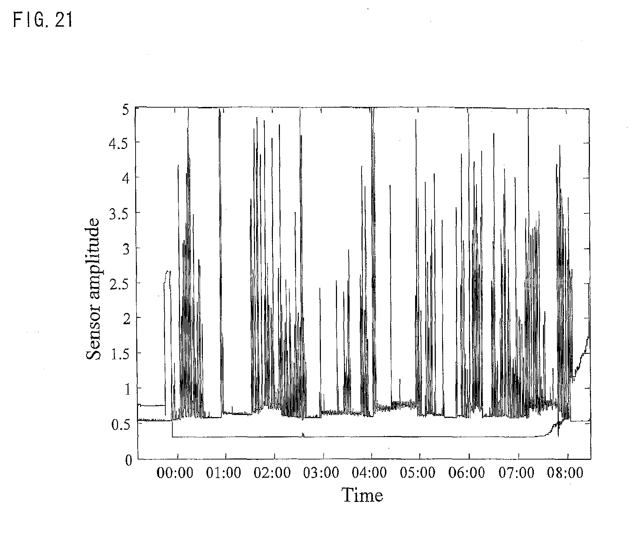

This example shows movement data, recorded using a piezoelectric cable, of an individual whilst going about their normal sleep routine. The piezoelectric cable was placed underneath the bed sheets on the user's bed. The data shows the user going to bed just before midnight, and getting out of bed shortly after 8am (Figure 21). The movement data (solid line) recorded shows periods where the user moved more frequently and periods where the user was more still. The movement data provides information on periods of light and deep sleep. Figure 21 also shows, plotted as a dashed line, the light intensity in the user's bedroom as recorded by a light sensor (eg. a photodiode). The output from the light sensor records the user turning off the bedroom lights shortly before midnight. The photodiode output also detects the room getting lighter due to sunrise, which in this instance started at approximately 07:30am. A further spike in the light trace is recorded shortly after 8am and corresponds to the user turning on their bedroom light as they rose from bed.

In the embodiments of the present invention, a "combined sleep indicator metric" is generated from at least one cognitive and / or psychomotor performance metric and at least one sleep metric and/or at least one sleep quality metric (with the sleep metric(s) and/or sleep quality metric(s) being determined from the sensor unit data). In the general case, this combined sleep indicator metric is calculated as a function of any number of sleep metrics, sleep quality metrics and cognitive and / or psychomotor performance metrics:

In some embodiments, combined sleep indicator metric = f(sleep metrics, sleep quality metrics, cognitive and / or performance metrics).

In embodiments that make use of the combined sleep indicator metric, the combined sleep indicator metric thus enables both information about the user's sleep and information about the user's cognitive and / or psychomotor performance to be combined in a single metric. Alternatively, in other embodiments at least one cognitive and/ or psychomotor performance metric together with at least one sleep metric or sleep quality metric may be determined.

In some embodiments, the data processing to generate the metric(s) may take place wholly in one processing means. Alternatively, the sensor data may undergo some processing at the sensor unit before being transmitted to a processing means for determination of the metric(s). As a further alternative the sensor data may be processed at the sensor unit to generate at least one sleep metric and/or at least one sleep quality metric, which is then transmitted to the processing means - for example, the processing means may then use a received sleep quality metric or sleep metric as input for the generation of the combined sleep indicator metric, or may simply generate the cognitive and/ or psychomotor performance metric.

In some embodiments, the information relating to the user's sleep behaviour that is sent from the processing means to the portable user interaction device for display to the user may as one example comprise the metric(s). Alternatively, the information may comprise information derived from the metric(s), but not the metric(s) themselves. As a further example, the information may additionally or alternatively comprise some or all of the original data collected by the sensor unit and/or input to the portable user interaction device.

In some embodiments, preferably, the one or more sensors do not include any sensor attached to the user.

Some embodiments may further comprise supplementing the objective test data with subjective feedback from the user on sleep-related parameters inputted via the user interaction device.

Some embodiments may further comprise displaying information to the user by the user interaction device, the information including one or more recommendations for affecting the behaviour of the user in order to improve subsequent sleep quality. The one or more recommendations may be selected from recommendations including behavioural programs and/or actions by the user.

Some embodiments may further comprise prompting the user, via the portable user interaction device, to implement a behavioural change.

In some embodiments, the processing means may monitor efficacy of and /or compliance with any behavioural program or action presented to and chosen by the user and provide (i) one or more of warning, guidance, advice and message(s) of encouragement to the user to aid efficacy of and/or compliance with any selected program or action and /or (ii) one or more updated recommendations for behavioural changes and / or actions.

Some embodiments may further comprise repeating steps (i) to (iv) at a subsequent time thereby to generate a combined sleep indicator metric for the subsequent time, or a cognitive and/ or psychomotor performance metric together with a sleep metric or a sleep quality metric for the subsequent time. By generating the metric(s) at one or more subsequent times, the user is able to see, from the scores of the metric(s), whether any behavioural changes they have made have lead to an improvement in their sleep quality.

In some embodiments, cognitive and / or psychomotor performance may be measured through presentation to the user via the portable user interaction device of one or more tests selected from mathematical processing, logical reasoning, spatial processing, reaction time, tracking, attention/ vigilance, self-generation cognitive function tests and memory tests.

In some embodiments, the processing means may receive data indicative of cognitive and / or psychomotor performance derived from passive monitoring of an activity of the user when awake, the data being obtained from the portable user interaction device or an additional source.

In some embodiments, the processing means may receive data on speed and / or accuracy of typing at a keyboard or keypad provided by the portable user interaction device or separately therefrom.

In some embodiments, the sensor unit may contain a memory and a real time clock whereby each sensor reading or multiple of sensor readings from each sensor for storage in the memory may be stored with a time code and sensor data stored in the memory is sent, for example periodically, sporadically, or on request, to the processing means.

Some embodiments may further comprise monitoring, using a plurality of sensors, both physiological and environmental parameters non-obtrusively such that they do not affect the sleep of the user.

In some embodiments, the physiological and environmental parameters monitored by the sensors may include movement and one or more of temperature, ambient noise, light and humidity.

In some embodiments, the at least one sensor may comprise a movement sensor which comprises a piezoelectric sheet, cable or film disposed below the user in bed and connected to the sensor unit via a cable or wireless connection.

Some embodiments may further comprise sampling, within the sensor unit, signal data from each sensor at a frequency of about 1 Hz before storage to a/the memory. For example, the signal data may be sampled at a frequency in the range of 0.1 Hz to 100Hz.

Some embodiments may be for use by the user and a second user who share a bed, and a second portable user interaction device, preferably identical to the portable user interaction device, may be provided for the second user, the second portable user interaction device communicating data to the processing means and receiving information from the processing means.

In some embodiments, the or each portable user interaction device may be a mobile phone.

Some embodiments may further comprise the or each portable user interaction device prompting the user to input information.

In some embodiments, the processing means may be separate from the sensor unit and the portable user interaction device. Alternatively, the processing means takes the form of a software service connected to a wide-area network such as, for example, the Internet.

In some embodiments, preferably, the sensor(s) employed will not include any sensor attached to the user.

In some embodiments, the objective test data may be supplemented with subjective feedback from the user on sleep-related parameters inputted via the user interaction device.

In some embodiments, the information output displayed to the user by the user interaction device may include one or more recommendations generated by the processing means to affect the behaviour of the user in order to improve subsequent sleep quality. The one or more recommendations may be selected from recommendations including behavioural programs and/ or actions by the user. Generally, the processing means will additionally provide one or more recommendations via the portable user interaction device to guide the behaviour of the user with the aim of improving subsequent sleep quality. Such recommendations may be selected from recommendations including behavioural programs and / or actions of the user. The system will desirably be able to monitor compliance with any such recommendation and update the recommendation(s) on the basis of subsequent correlation by the processing means of sensor data and objective test data indicative of cognitive and/ or psychomotor performance. The portable user interaction device may have an alerting function to prompt the user to carry out action(s) required to implement a behavioural change and/ or input information.

In some embodiments, the portable user interaction device may have an alerting function to prompt the user to implement a recommended behavioural change, for example by prompting the user to carry out one or more actions required to implement a behavioural change.

In some embodiments, the processing means may monitor efficacy of and /or compliance with any behavioural program or action presented to and chosen by the user and provide (i) one or more of warning, guidance, advice and message(s) of encouragement to the user to aid efficacy of and/or compliance with any chosen program or action and /or (ii) one or more updated recommendations for behavioural changes and / or actions.

In some embodiments, cognitive and / or psychomotor performance may be measured through presentation to the user via the portable user interaction device of one or more tests selected from mathematical processing, logical reasoning, spatial processing, reaction time, tracking, attention/ vigilance, self-generation cognitive function tests and memory tests.

In some embodiments, the processing means may receive data indicative of cognitive and / or psychomotor performance derived from passive monitoring of an activity of the user when awake, the data being obtained from the portable user interaction device or from an additional source.

In some embodiments, the processing means may receive data on speed and / or accuracy of typing at a keyboard or keypad provided by the portable user interaction device or provided separately therefrom.

In some embodiments, the sensor unit may contain a memory and a real time clock whereby each sensor reading or multiple of sensor readings from each sensor for storage in the memory is stored with a time code and sensor data stored in the memory is sent periodically, or on request, to the processing means.

In some embodiments, the system may comprise a plurality of sensors for monitoring both physiological and environmental parameters non-obtrusively such that they do not affect the sleep of the user. The physiological and environmental parameters monitored by the sensors may include movement and one or more of temperature, ambient noise, light and humidity.

In some embodiments, the at least one sensor may comprise a movement sensor which comprises a piezoelectric sheet, cable or film disposed below the user in bed and connected to the sensor unit via a cable or wireless connection.

In some embodiments, within the sensor unit signal data from each sensor may be sampled by a processor at a frequency of about 1Hz before storage to a/the memory. For example, the signal data may be sampled at a frequency in the range of 0.1 Hz to 100Hz.

In some embodiments, the system may be for use by the user and a second user who share a bed, wherein an additional portable user interaction device identical to the portable user interaction device may be provided for the second user, the additional portable user interaction device being adapted to communicate data to the processing means and receive information from the processing means.

In some embodiments, the or each portable user interaction device may be a mobile phone.

In some embodiments, the or each portable user interaction device may have an alerting function to prompt the user to input information.

In some embodiments, the processing means may be separate from the sensor unit and the portable user interaction device.

In some embodiments, the processing means may take the form of a software service connected to a wide-area network such as the Internet.

Another aspect of the invention provides a method of managing sleep of an individual which comprises the individual using a sleep management system of the second aspect.

Since, as noted above, circadian rhythm may impact on cognitive and/or psychomotor performance depending on the time of day test data is collected by the portable user interaction device, e.g. by the user carrying out a psychomotor vigilance task (PVT) presented by the portable device display, then implementation of the sleep management system may desirably take account of this. Accordingly, the processing means may take account of circadian rhythm in analysing objective test data from the portable user interaction device by (a) and /or one of (b) and (c) as follows: (a) the processing means having available one or more pre-defined functions for cognitive and/or psychomotor performance from pre-collected data on variance of test performance of individuals with time of day; (b) the user initially after practising one or more cognitive and / or psychomotor tests on the portable user interaction device, carrying out the same test(s) at a range of times and over a time period whereby the processing means can establish a baseline for cognitive and / or psychomotor performance over the user's wake hours, or (c) the user initially carrying out one or more cognitive and/ or psychomotor tests on the portable user interaction device at a range of times and over a period whereby the processing means can establish a baseline for cognitive and /or psychomotor performance during the time the user is awake relying on a pre-defined function to correct for effects due to the user practicing.

Prior to use in improving sleep quality, the processing means may be provided with information to take account of circadian rhythm in analysing objective test data from the portable user interaction device by (a) and/or one of (b) and (c) as follows: (a) providing the processing means with one or more pre-defined functions for cognitive and/or psychomotor performance from pre-collected data on variance of test performance of individuals with time of day; (b) after practising one or more cognitive and / or psychomotor tests on the portable user interaction device, the user carrying out the same test(s) at a range of times and over a time period whereby the processing means can establish a baseline for cognitive and / or psychomotor performance over the user's wake hours, or (c) the user carrying out one or more cognitive and/ or psychomotor tests on the portable user interaction device at a range of times and over a period whereby the processing means can establish a baseline for cognitive and /or psychomotor performance during the time the user is awake relying on a pre-defined function to correct for effects due to the user practicing.

In some embodiments, the portable user interaction device may prompt the user to carry out the one or more tests at pre-defined times, sporadic times or random times during the time the user is awake.

In some embodiments, the processing means may generate a cognitive and/ or psychomotor performance metric and a sleep metric or a sleep quality metric. Alternatively, the sleep metric or sleep quality metric may be generated elsewhere and passed to the processing means, in which case the processing means need generate only a cognitive and/ or psychomotor performance metric (or a combined sleep indicator metric).

In some embodiments, the portable device may prompt the user to carry out any suggested test at pre-defined times or random times during wake hours to achieve an acceptable performance baseline stored in a memory of the processing means. This may be when the stored performance data does not exceed a pre-determined variability.

In some embodiments, it will be recognized that in use a system of the invention may recognize a sleep problem requiring specialist, even medical treatment, in which case a recommendation will be provided to seek specialist advice, e. g. from a doctor. However, it will be appreciated that the portable user interaction device element of the system can only impart information to the user and encourage behavioural action, which might include seeking medical treatment for an underlying medical problem affecting sleep, but cannot provide treatment per se.

In some embodiments, a sleep management system of the invention may be implemented as a combination of hardware units which comprises (i) the required sensor(s) (2a-e) (ii) the sensor unit (1) and (iii) the portable user interaction device(s) (8) which can collect objective test data periodically or sporadically from the user when awake indicative of cognitive and/or psychomotor performance.

In some embodiments, the or each portable user interaction device may be able to present to the user one or more tests selected from mathematical processing, logical reasoning, spatial processing, reaction time, tracking, attention/ vigilance, self-generation cognitive function tests, memory tests.

In some embodiments, the or each portable user interaction device may be able to passively monitor an activity of the user when awake indicative of cognitive and/ or psychomotor performance.

In some embodiments, the or each portable user interaction device may be able to provide a keyboard or keypad whereby speed and /or accuracy of typing can be monitored.

In some embodiments, the or each portable user interaction device may be a mobile phone.

In some embodiments, a fifth aspect of the invention provides a computer-readable medium containing instructions that, when executed by a processor, cause the processor to perform a method of the invention.

**References**

1. The following documents describe systems that monitor and evaluate sleep quality:

- EP 1 810 710 A1, 14.09.2005, Junichiro Arai, "Sleeping state improving system, and sleeping state improving method".
- US 5,229,428, 05.04.1994, Mariko Kawaguri et al, "Environmental control system and method".
- US 6,468,234 B1, 10.11.2000 (Provisional application 60/218,238 filed Jul 14 2000), H. F. Machiel Van der Loos et al, "Sleepsmart".
- US 6,485,411 B2, 17.01.2002, (Provisional application 60/218,238 filed Jul 14 2000), Steven H Woodward, "Sensorbed".
- US 7,179,218 B2, 18.05.2006, (Division of application No 10/718,960 21.11.2003 now patent 7,041,049), Keith Raniere, "Sleep Guidance system and related methods".
- US 5,479,939, 06.04.1994, Hiroyuki Ogina, Matsushita Electric Industrial Co., "Sleep detecting apparatus"
- US 2004/0087878 A1, 01.11.2002, David T. Krausman et al, "Sleep scoring apparatus and method".
- US 2005/0042589 A1, 18.08.2003, John D Hatlestad et al, "Sleep quality data collection and evaluation".
- US 2005/0096559 A1, 21.09.2004 (Foreign application priority date 29.10.2003 JP 2003-369366), Kenichi Yanai, "Sensor sheet".
- US 2005/0143617 A1, 29.12.2004 (Provisional application 60/534,168 filed 31.12.2003), Raphael Auphan, "Sleep and environment control method and system".
- US 2005/0222522 A1, 16.03.2005 (Provisional application 60/553,771 filed 16.03.2004), Kenneth T. Heruth, "Detecting sleep".
- US 2006/0135854 A9, 16.05.2003 (Provisional application 16.05.2002), Sean P. McDonough *et al*, "Sleep study software architecture".
- US 2006/0224047 A1, 23.11.2005 (Foreign application priority date 30.03.2005), Takuji Suzuki *et al,* "Sleepiness prediction apparatus and sleepiness prediction method."
- US 2006/0293602 A1, 08.04.2004 (Foreign application priority date 16.04.2003), Richard Charles Clark, "Sleep management device".
- US 2006/0293608 A1, 28.02.2005 (Provisional application 27.02.2004), Daniel Rothman *et al,* "Device for and method of predicting a user's sleep state".
- US 2007/0191692 A1, 18.10.2006, (Foreign application priority date 14.02.2006), Yeh-Liang Hsu *et al,* "Sleeping quality monitor system and method for monitoring a physiological signal".
- US 2008/0033304 A1, 19.07.2006, Yousufali Dalal et al, "Sleep state detection".
- US 7,366,572 B2, 15.04.2004, Kenneth T. Heruth et al, "Controlling therapy based on sleep quality".
- US 2004/0230398 A1, 13.05.2004 (Foreign application priority 15.05.2003), Shima Okada *et al,* "Sleep analyzer and program product for giving sleep analysis function to computer".
- US 2007/008307 A1, 31.08.2006, Mi-hee Lee et al, "Apparatus and/or method for inducing sound sleep and waking".
- US 2008/0157956 A1, 29.12.2006, Zoran Radivojevic et al, "Method for the monitoring of sleep using an electronic device".
- US 2008/0306351 A1, 06.06.2008, Shuichi Izumi, "Sleep evaluation device".
- WO 2004/075714 A3, 25.02.2004 (Priority 28.02.2003), Cornel Lustig, "Device for manipulating the state of alertness".

- WO 2005/066868 A2, 31.12.2004 (Priority 31.12.2003), Raphael Auphan, "Sleep and environment control method and system".
- WO 2005/089641 A1, 16.03.2005 (Priority 15.04.2004), Keith A. Miesel *et al,* "Detecting sleep".
- WO 2004/075714 A2, 25.02.2004, Cornel Lustig, "System and method for manipulating sleep architecture by sub-awakening non-intrusive stimulations".
- WO 2008/037020 A1, 27.09.2007, Frances Renee Doherty et al, "Methods and apparatus for assessing sleep quality".

2. The following documents describe some common sleep disorders and discuss how they can impact on an individual's quality of life:

- Socioeconomic impact of insomnia in working populations, Industrial Health, 2005, 43,11
- Evaluation of Quality of Life in Severe and Mild Insomniacs Compared With Good Sleepers, Psychosomatic Medicine, 2001, 63, 49
- Epidemiology of Insomnia, Depression, and Anxiety, Sleep, 2005, 11, 1457
- The Cost of Sleep-Related Accidents, Sleep, 1994, 17, 84
- The Direct and Indirect Costs of Untreated Insomnia in Adults in the United States, Sleep, 2007, 30, 263
- The economic cost of sleep disorders, Sleep, 2006, 29, 299-305.
- ABC of Sleep Disorders, ed. Colin M. Shapiro, BMJ 1993
- A new method for measuring daytime sleepiness: The Epworth sleepiness scale, Murray W. Johns, Sleep, 14 (6), 540-545 (1991).
- Insomnia is more common among subjects living in damp buildings, C. Janson et al, Occup Environ Med, 62, 113-118 (2005).
- Sleep Hygiene in Physically Dependent Nursing Home Residents: Behavioural and Environmental Intervention Implications, John F. Schnelle, Patrice A. Cruise, Cathy A. Alessi, Karen Ludlow, Nahla R. Al-Samarrai and Joseph G. Ouslander, Sleep, 21(5), 515 (1998).
- Restless legs syndrome and sleep bruxism: prevalence and association among Canadians, G. J. Lavigne and J. Y. Montplaisir, Sleep 17(8), 739-746 (1994).

3. Further information on how behavioural therapies can be used to improve sleep quality can be found in the following documents:

- EP 1 618 913 A1, 21.07.2005, Albert Behar et al, "Device for insomnia assessment and automated sleep behaviour modification".
- US 2004/0225179 A1, 01.03.2004, Richard Kaplan et al, "Automated insomnia treatment system".

4. Information on how cognitive performance is linked to sleepiness can be found in the following documents:

• The validity of psychomotor vigilance tasks of less than 10-minute duration, Behaviour Methods, Instruments and Computers, 36 (2), 339-346 (2004).
• Performance vigilance task and sleepiness in patients with sleep-disordered breathing, European Respiratory Journal, 24, 279-285 (2004).
• Effects Of Sleep Deprivation On Performance: A Meta-Analysis, Sleep, 1996, 19, 318
• Cumulative sleepiness, mood disturbance and psychomotor vigilance performance decrements during a week of sleep restricted to 4-5 hours per night, American Sleep Disorders Association and Sleep Research Society, 20(4), 267-277 (1997).
• 'Cognitive and psychomotor performance tests and experiment design in multiple chemical sensitivity', Anthony Wetherell, Environmental Health Perspectives, Vol 105, Supplement 2, March 1997

5. Further information on predicting human cognitive performance with regards to carrying out a particular task based on their sleep patterns can be found in the following documents:

- US 6,743,167 B2, 04.03.2003 (Prior publication 28.08.2003), Thomas J. Balkin *et al,* "Method and system for predicting human cognitive performance using data from an actigraph".
- US 7,207,938 B2, 11.01.2005, Steven Rawlings Hursh, "Evaluating task effectiveness based on sleep pattern".

6. The following documents describe how environmental parameters, such as temperature, noise and humidity, can affect sleep quality:

- Effects of humid heat exposure on human sleep stages and body temperature, Okamoto-Mizuno K, Mizuno K, Michie S, Maeda A, Iizuka S., Sleep, 22, 767 (1999).
- The effects of high and low ambient temperatures on human sleep stages, E. H. Haskell et al, Electroencephalography and Clinical Neurophysiology, 51, 494 - 501 (1981).
- Nighttime drop in body temperature: A physiological trigger for sleep onset?, Patricia J. Murphy and Scott S. Campbell, American Sleep Disorders Association and Sleep Research Society, 20 (7), 505-511 (1997).
- Influence of ambient temperature on sleep characteristics and autonomic nervous control in healthy infants, Franco P, Szliwowski H, Dramaix M, Kahn A., Sleep, 23, 401 (2000).
- Ambient temperature is associated with changes in infants' arousability from sleep, Franco P, Scaillet S, Valente Ir F, Chabanski S, Groswasser J, Kahn A., Sleep, 24, 325(2001).
- Interaction between sleep and thermoregulation: an aspect of the control of behavioural states, Parmeggiani PL., Sleep, 10, 426 (1987).
- Relative and combined effects of heat and noise exposure on sleep in humans, Libert JP, Bach V, Johnson LC, Ehrhart J, Wittersheim G, Keller D., Sleep, 14, 24 (1991).
- Influence of ambient temperature on sleep characteristics and autonomic nervous control in healthy infants, P. Franco, H. Szliwowski, M. Dramaix and A. Khan, Sleep, 23, 401 (2000).
- Temperature and air humidity as factors influencing sleep and wakefulness, D Svorad, J Wellnerovà International Journal of Biometeorology, 1959
- Relative and combined effects of heat and noise exposure on sleep in humans, J. P. Libert, V. Bach, L. C. Johnson, J. Ehrhart, G. Wittersheim and D. Keller, Sleep, 14(1), 24-31 (1991).
- Effects of aircraft noise on sleep: EEG-based measurements, Manchester Metropolitan University, K I Hume, F Van & A Watson, June 2003.
- A field study of sleep disturbances: Effects of aircraft noise and other factors on 5,742 nights of actimetrically monitored sleep in a large subject sample, J. A. Horne, F. L. Pankhurst, L. A. Reyner, K. Hume and I. D. Diamond, Sleep, 17(2), 146-159 (1994).
- Sleep disturbances due to exposure to tone pulses throughout the night, Yasuaki Nakagawa, Sleep, 10(5), 463-472 (1987).
- Environmental Noise As A Cause Of Sleep Disruption In An Intermediate Respiratory Care Unit, Joshua N. Aaron, Carol C. Carlisle, Mary A. Carskadon, Thomas J. Meyer, Nicholas S. Hill and Richard P. Millman, Sleep, 19(9), 707 (1996).
- Environmental Noise And Sleep - A Study Of Arousals, Cardiac Arrhythmia And Urinary Catecholamines, N.L. Carter, S.N. Hunyor, G. Crawford, D. Kelly and A.J.M. Smith, Sleep, 17(4), 298 (1994).

7. Further information on the science of sleep and the techniques typically used by qualified sleep professionals can be found in the following references:

• Actigraphically recorded motor activity and immobility across sleep cycles and stages in healthy male subjects, Middelkoop HA, Van Hilten BJ, Kramer CG, Kamphuisen HA. J Sleep Res. 2(1), 28 (1993).
• 'Principles and practice of sleep medicine, 4th edition', Meir H. Kryger, Thomas Roth, William C. Dement, Elsevier Saunders ISBN 0-7216-0797-7.

Although the invention has been shown and described with respect to certain preferred embodiments, it is obvious that equivalents and modifications will occur to others skilled in the art upon the reading and understanding of the specification. The present invention includes all such equivalents and modifications, and is limited only by the scope of the following claims.

**Claims**

1.  A method of managing the sleep of a user, the method comprising:

(i) monitoring, using at least one sensor (2a-2e), one or more objective parameters relevant to sleep quality of the user when in bed, said parameters being selected from physiological parameters and environmental parameters and including at least movement of the user and /or electrical signals indicative of brain activity of the user;
(ii) collecting, using a sensor unit (1), signal data from said at least one sensor and communicating the signal data to a processing means (16);

(iii) collecting, using a portable user interaction device (8), objective test data from the user when awake, the objective test data being indicative of cognitive and/or psychomotor performance and communicating the objective test data to said processing means; and

(iv) processing data from said sensor unit and said user interaction device to generate at least a combined sleep indicator metric, which takes both sensor unit data and cognitive and/or psychomotor data into consideration, or a cognitive and/ or psychomotor performance metric together with a sleep metric or a sleep quality metric; and

(v) receiving at the portable user interaction device (8) information relating to the user's sleep behaviour for display to the user.

2. A method according to claim 1 wherein said at least one sensor does not include any sensor attached to the user; and/or comprising supplementing said objective test data with subjective feedback from the user on sleep-related parameters inputted via said user interaction device.

3. A method as claimed in any one of claims 1 to 2 further comprising displaying information to the user by the user interaction device, the information including one or more recommendations for affecting the behaviour of the user in order to improve subsequent sleep quality.

4. A method as claimed in claim 3 wherein said one or more recommendations are selected from a group of recommendations including behavioural programs and/ or actions by the user; and optionally wherein the method further comprises prompting the user, via said portable user interaction device, to implement a recommended behavioural change and/or wherein said processing means monitors efficacy of and /or compliance with any behavioural program or action presented to and chosen by the user and provides (i) one or more of warning(s), guidance, advice and message(s) of encouragement to the user to aid efficacy of and/or compliance with any selected program or action and /or (ii) one or more updated recommendations for behavioural changes and / or actions.

5. A method as claimed in any preceding claim and comprising repeating steps (i) to (iv) at a subsequent time thereby to generate a combined sleep indicator metric for the subsequent time, or a cognitive and/ or psychomotor performance metric together with a sleep metric or sleep quality metric for the subsequent time and/or wherein cognitive and / or psychomotor performance is measured through presentation to the user via said portable user interaction device of one or more tests selected from mathematical processing, logical reasoning, spatial processing, reaction time, tracking, attention/ vigilance, self-generation cognitive function tests and memory tests.

6. A method as claimed in any one of claims 1 to 5 wherein said processing means receives data indicative of cognitive and / or psychomotor performance derived from passive monitoring of an activity of the user when awake, said data being obtained from said portable user interaction device or an additional source, and optionally wherein said processing means receives data on speed and / or accuracy of typing at a keyboard or keypad provided by said portable user interaction device or separately therefrom.

7. A method as claimed in any one of the preceding claims wherein said sensor unit (1) contains a memory (5) and a real time clock (4) whereby each sensor reading or multiple of sensor readings from each sensor for storage in the memory is stored with a time code and sensor data stored in said memory is sent periodically, or on request, to said processing means (16).

8. A method as claimed in any one of the preceding claims, comprising monitoring, using a plurality of sensors (2a-e), both physiological and environmental parameters non-obtrusively such that they do not affect the sleep of the user, and optionally wherein the physiological and environmental parameters monitored by said sensors include movement and one or more of temperature, ambient noise, light and humidity.

9. A method as claimed in any one of the preceding claims wherein the at least one sensor comprises a movement sensor which comprises a piezoelectric sheet, cable or film disposed below the user in bed and connected to the sensor unit via a cable or wireless connection; and/or wherein the method comprises sampling, within the sensor unit (1), signal data from each sensor at a frequency in the range of 0.1 Hz to 100Hz before storage to a/the memory (5); and/or wherein the or each portable user interaction device is a mobile phone; and/or wherein the method further comprises the or each portable user interaction device prompting the user to input information.

10. A method as claimed in any one of the preceding claims for use by the user and a second user who share a bed, wherein a second portable user interaction device identical to the portable user interaction device is provided for the second user, the second portable user interaction device communicating data to said processing means and receiving information from the processing means.

**11.** A method as claimed in any one of the preceding claims wherein said processing means is separate from said sensor unit and said portable user interaction device, and optionally wherein said processing means takes the form of a software service connected to a wide-area network.

**12.** A sleep management system comprising:

(i) at least one sensor (2a-2e) for monitoring one or more objective parameters relevant to sleep quality of the user when in bed, said parameters being selected from physiological parameters and environmental parameters and including at least movement of the user and /or electrical signals indicative of brain activity of the user;
(ii) a sensor unit (1) which collects signal data from said at least one sensor and communicates data to a processing means (16);
(iii) a portable user interaction device (8), which can collect objective test data from the user when awake indicative of cognitive and/or psychomotor performance and which additionally communicates data to said processing means and receives information from the processing means which is displayed to the user, and
(iv) said processing means, which processes data from said sensor unit and said portable user interaction device whereby at least a combined sleep indicator metric, which takes both sensor unit data and cognitive and/or psychomotor data into consideration, or a cognitive and/ or psychomotor performance metric together with a sleep metric or a sleep quality metric can be displayed to the user via the portable user interaction device.

**13.** A method as claimed in any of claims 1 to 11 wherein said processing means can take account of circadian rhythm in analysing objective test data from said portable user interaction device by (a) and /or one of (b) and (c) as follows:

(a) said processing means having available one or more pre-defined functions for cognitive and/or psychomotor performance from pre-collected data on variance of test performance of individuals with time of day;
(b) the user initially after practising one or more cognitive and / or psychomotor tests on said portable user interaction device, carrying out the same test(s) at a range of times and over a time period whereby said processing means can establish a baseline for cognitive and / or psychomotor performance over the user's wake hours, or
(c) the user initially carrying out one or more cognitive and/ or psychomotor tests on said portable user interaction device at a range of times and over a period whereby said processing means can establish a baseline for cognitive and /or psychomotor performance during the time the user is awake relying on a pre-defined function to correct for effects due to the user practicing;
and/or wherein, prior to use in improving sleep quality, said processing means is provided with information to take account of circadian rhythm in analysing objective test data from said portable user interaction device by (a) and/or one of (b) and (c) as follows:

(a) providing said processing means with one or more pre-defined functions for cognitive and/or psychomotor performance from pre-collected data on variance of test performance of individuals with time of day;
(b) after practising one or more cognitive and / or psychomotor tests on said portable user interaction device, the user carrying out the same test(s) at a range of times and over a time period whereby said processing means can establish a baseline for cognitive and / or psychomotor performance over the user's wake hours, or
(c) the user carrying out one or more cognitive and/ or psychomotor tests on said portable user interaction device at a range of times and over a period whereby said processing means can establish a baseline for cognitive and /or psychomotor performance during the time the user is awake relying on a pre-defined function to correct for effects due to the user practicing.

**14.** A method as claimed in any one of claims 1 to 11 or 13 wherein the portable user interaction device prompts the user to carry out said one or more cognitive and/or psychomotor tests at pre-defined times or random times during the time the user is awake.

**15.** A computer-readable medium containing instructions that, when executed by a processor, cause the processor to perform a method as defined in any one of claims 1 to 11 and 13.

FIG. 1

FIG. 2

9    8    13  15

| Lifestyle Data | Screen | Speaker |
| --- | --- | --- |
| Sleep questionnaires Diet and exercise Perceived stress | | Battery / Vibrate unit |

Cognitive Data — Reaction time tests Logical reasoning Mathematical tests

User input keypad

Memory — 11

Network connection — 12

Portable Sensor Data — Temperature Noise Accelerometer

10

FIG. 3

17    16

Processor

Network connection — 19

Memory

18

20

FIG. 4

Processing
unit

Sensor
unit

Portable
unit

FIG. 5

Processing
unit

Sensor
unit

Portable
unit

Other
display unit
e.g. laptop

EP 2 278 508 A1

FIG. 6

```
┌─────────────────┐
│   Microphone    │
└────────┬────────┘
         ▼
┌─────────────────┐
│  Amplification  │
│   and filtering │
└────────┬────────┘
         ▼
┌─────────────────┐
│  Rectification  │
└────────┬────────┘
         ▼
┌─────────────────┐
│   Integration   │
└────────┬────────┘
         ▼
┌─────────────────┐
│      ADC        │
└────────┬────────┘
         ▼
┌─────────────────┐
│   Processor     │
└────────┬────────┘
         ▼
┌─────────────────┐
│     Memory      │
└─────────────────┘
```

FIG. 7

```
        ┌─────────────────────┐
        │    Piezoelectric    │
        │      element        │
        └──────────┬──────────┘
                   │
                   ▼
        ┌─────────────────────┐
        │   Amplification     │
        │   and filtering     │
        └──────────┬──────────┘
                   │
                   ▼
        ┌─────────────────────┐
        │                     │
        │    Rectification    │
        │                     │
        └──────────┬──────────┘
                   │
                   ▼
        ┌─────────────────────┐
        │                     │
        │    Integration      │
        │                     │
        └──────────┬──────────┘
                   │
                   ▼
        ┌─────────────────────┐
        │                     │
        │        ADC          │
        │                     │
        └──────────┬──────────┘
                   │
                   ▼
        ┌─────────────────────┐
        │                     │
        │     Processor       │
        │                     │
        └──────────┬──────────┘
                   │
                   ▼
        ┌─────────────────────┐
        │                     │
        │      Memory         │
        │                     │
        └─────────────────────┘
```

FIG. 8

1. How well do you think you slept last night?

○   ○   .○   ○   ○   ○   ○   ○
Very                                    Very
poorly                                  well

2. How refreshed do you feel today?

○   ○   ○   ○   ○   ○   ○   ○
Not                                     Very
refreshed                               refreshed

FIG. 9

1. How much caffeine did you consume yesterday?

0 cups        ○
1-5 cups      ○
5-10 cups     ○
>10 cups      ○

2. How much alcohol did you consume yesterday?

0 units       ○
1-2 units     ○
2-5 units     ○
>5 units      ○

FIG. 10

1. How do you feel this morning?

Tired                                                                 Alert

2. How stressed do you feel today?

Very                                                                 Not
stressed                                                         stressed

FIG. 11 (a)                                    FIG. 11 (b)

S1110
User carries out cognitive task(s) periodically whilst awake

S1120
Cognitive baseline established?  no

yes

S1130
Proceed to full system use, modes I - V

S1140
User carries out cognitive task(s) randomly whilst awake

S1120
Cognitive baseline established?  no

yes

S1130
Proceed to full system use, modes I - V

FIG. 12 (a)

S1210

User practices
cognitive task(s)
periodically whilst
awake

S1220

Asymptotic
level reached?        no

yes

S1230

Proceed to
determining
cognitive baseline

FIG. 12 (b)

S1240

User practices
cognitive task(s)
randomly whilst
awake

S1220

Asymptotic
level reached?        no

yes

S1230

Proceed to
determining
cognitive baseline

FIG. 12 (c)

S1250

User practices
cognitive task(s)
whilst awake

S1220

Asymptotic
level reached?        no

yes

S1260

Account for this
using a practice
effect function $f(p_e)$

S1230

Proceed to
determining
cognitive baseline

S1270

Proceed to determining
cognitive baseline
Proceed to full system use,
modes I - V

41

**FIG. 13**

```
                          ┌─ S1310
        ┌──────────────────────────┐          Mode I
        │   Sensors monitor sleep   │◄────────┐
        │      parameters non-      │         │
        │  invasively whilst user is │        │
        │        sleeping           │         │
        └──────────────┬───────────┘          │
                       │                       │
                       ▼  ┌─ S1320             │
        ┌──────────────────────────┐          │
        │      User provides        │          │
        │   subjective feedback     │          │
        │       when awake          │          │
        └──────────────┬───────────┘          │
                       │                       │
                       ▼  ┌─ S1330             │
        ┌──────────────────────────┐          │
        │   Objective measures of   │          │
        │        sleepiness are     │          │
        │   assessed whilst user is │          │
        │          awake            │          │
        └──────────────┬───────────┘          │
                       │                       │
                       ▼  ┌─ S1340             │
        ┌──────────────────────────┐          │
        │   Software determines     │          │
        │    sleep metrics using    │          │
        │      subjective and       │          │
        │   objective parameters    │          │
        └──────────────┬───────────┘          │
                       │                       │
                       ▼  ┌─ S1350             │
        ┌──────────────────────────┐          │
        │   Results from subjective │          │
        │      and objective        │──────────┘
        │        sleepiness         │
        │   measurements are        │
        │    presented to user      │
        └──────────────────────────┘
```

FIG. 14

```
                                            ┌─S1410
                  ┌──────────────────────┐            Mode II
                  │  Sensors monitor sleep │
                  │    parameters non-     │◄───────────┐
                  │ invasively whilst user is │         │
                  │       sleeping         │            │
                  └──────────────────────┘            │
                            │                          │
                            ▼       ┌─S1420            │
                  ┌──────────────────────┐            │
                  │    User provides       │            │
                  │  subjective feedback   │            │
                  │     when awake         │            │
                  └──────────────────────┘            │
                            │                          │
                            ▼       ┌─S1430            │
                  ┌──────────────────────┐            │
                  │  Objective measures of │            │
                  │    sleepiness are      │            │
                  │  assessed whilst user is │          │
                  │        awake           │            │
                  └──────────────────────┘            │
                            │                          │
                            ▼       ┌─S1440            │
                  ┌──────────────────────┐            │
                  │  Software determines   │            │
                  │   sleep metrics using  │            │
                  │     subjective and     │            │
                  │  objective parameters  │            │
                  └──────────────────────┘            │
                            │                          │
                            ▼       ┌─S1450            │
                  ┌──────────────────────┐            │
                  │ Results from subjective │           │
                  │    and objective       │            │
                  │     sleepiness         │            │
                  │  measurements are      │────────────┘
                  │   correlated and       │
                  │  presented to user     │
                  └──────────────────────┘
```

FIG. 15

Mode III

```
                          ┌─S1410
        ┌──────────────────────┐
        │   Sensors monitor sleep  │◄─────┐
        │    parameters non-       │      │
        │  invasively whilst user is│      │
        │       sleeping           │      │
        └──────────┬───────────┘      │
                   │  ┌─S1420            │
        ┌──────────▼───────────┐      │
        │     User provides        │      │
        │   subjective feedback    │      │
        │       when awake         │      │
        └──────────┬───────────┘      │
                   │  ┌─S1430            │
        ┌──────────▼───────────┐      │
        │   Objective measures of  │      │
        │      sleepiness are      │      │
        │   assessed whilst user is│      │
        │          awake           │      │
        └──────────┬───────────┘      │
                   │  ┌─S1440            │
        ┌──────────▼───────────┐      │
        │   Software determines    │      │
        │    sleep metrics using   │      │
        │      subjective and      │      │
        │   objective parameters   │      │
        └──────────┬───────────┘      │
                   │  ┌─S1450            │
        ┌──────────▼───────────┐      │
        │ Results from subjective  │      │
        │       and objective      │      │
        │        sleepiness        │      │
        │     measurements are     │      │
        │       correlated and     │      │
        │      presented to user   │      │
        └──────────┬───────────┘      │
                   │  ┌─S1510            │
        ┌──────────▼───────────┐      │
        │    User can access       │      │
        │  information which will  │      │
        │    enable them to        │      │
        │    condition their       │──────┘
        │  behaviour to improve    │
        │  their quality of sleep  │
        └──────────────────────┘
```

FIG. 16

```
                                        S1410
        ┌─────────────────────────┐              Mode IV
        │ Sensors monitor sleep   │
        │ parameters non-         │◄────────┐
        │ invasively whilst user is│         │
        │ sleeping                │         │
        └─────────────────────────┘         │
                    │                       │
                    ▼      S1420            │
        ┌─────────────────────────┐         │
        │ User provides           │         │
        │ subjective feedback     │         │
        │ when awake              │         │
        └─────────────────────────┘         │
                    │                       │
                    ▼      S1430            │
        ┌─────────────────────────┐         │
        │ Objective measures of   │         │
        │ sleepiness are          │         │
        │ assessed whilst user is │         │
        │ awake                   │         │
        └─────────────────────────┘         │
                    │                       │
                    ▼      S1440            │
        ┌─────────────────────────┐         │
        │ Software determines     │         │
        │ sleep metrics using     │         │
        │ subjective and          │         │
        │ objective parameters    │         │
        └─────────────────────────┘         │
                    │                       │
                    ▼      S1450            │
        ┌─────────────────────────┐         │
        │ Results from subjective │         │
        │ and objective           │         │
        │ sleepiness              │         │
        │ measurements are        │         │
        │ correlated and          │         │
        │ presented to user       │         │
        └─────────────────────────┘         │
                    │                       │
                    ▼      S1460            │
        ┌─────────────────────────┐         │
        │ User can access         │         │
        │ information which will  │         │
        │ enable them to          │         │
        │ condition their         │         │
        │ behaviour to improve    │         │
        │ their quality of sleep  │         │
        └─────────────────────────┘         │
                    │                       │
                    ▼      S1610            │
        ┌─────────────────────────┐         │
        │ User can utilise        │         │
        │ portable device in order│         │
        │ to assist with desired  │─────────┘
        │ behavioural             │
        │ conditioning            │
        └─────────────────────────┘
```

## FIG. 17

```
                                    ┌─S1410
        ┌──────────────────────┐
        │  Sensors monitor sleep │          Mode V
        │   parameters non-      │◄──────────┐
        │ invasively whilst user is│         │
        │      sleeping          │           │
        └──────────┬───────────┘             │
                   │  ┌─S1420                 │
        ┌──────────▼───────────┐             │
        │    User provides      │            │
        │  subjective feedback  │            │
        │     when awake        │            │
        └──────────┬───────────┘             │
                   │  ┌─S1430                 │
        ┌──────────▼───────────┐             │
        │ Objective measures of │            │
        │    sleepiness are     │            │
        │ assessed whilst user is│           │
        │       awake           │            │
        └──────────┬───────────┘             │
                   │  ┌─S1440                 │
        ┌──────────▼───────────┐             │
        │ Software determines   │            │
        │  sleep metrics using  │            │
        │    subjective and     │            │
        │ objective parameters  │            │
        └──────────┬───────────┘             │
                   │  ┌─S1450                 │
        ┌──────────▼───────────┐             │
        │ Results from subjective│           │
        │     and objective     │            │
        │      sleepiness       │            │
        │   measurements are    │            │
        │    correlated and     │            │
        │   presented to user   │            │
        └──────────┬───────────┘             │
                   │  ┌─S1710                 │
        ┌──────────▼───────────┐             │
        │  Only unusual or      │            │
        │ significant changes in │           │
        │   sleep metrics are   ├────────────┘
        │   presented to user   │
        └──────────────────────┘
```

FIG. 18

```
┌─────────────────────────┐
│      Set-up system      │
└─────────────────────────┘
             │
             ▼
┌─────────────────────────┐
│ Establish baseline dataset│
└─────────────────────────┘
             │
             ▼
┌─────────────────────────┐
│  Analyse data for baseline│
│          period         │
└─────────────────────────┘
             │
             ▼
┌─────────────────────────┐
│   Establish correlations │
│   between objective and  │
│  subjective information for│
│     baseline dataset     │
└─────────────────────────┘
             │
             ▼
┌─────────────────────────┐
│    Report back to user   │
└─────────────────────────┘
             │
             ▼
       ╱──────────╲        yes    ┌──────────────────────────────┐
      ╱ Behavioural ╲──────────────│  Record behavioural change   │◄───┐
      ╲   change?   ╱              └──────────────────────────────┘    │
       ╲──────────╱                            │                       │
          no │                                 ▼                       │
             ▼                    ┌──────────────────────────────┐     │
┌─────────────────────────┐      │     Establish new dataset    │     │
│ Continue in mode I, II, III or V│      └──────────────────────────────┘     │
└─────────────────────────┘                    │                       │
                                               ▼                       │
                                  ┌──────────────────────────────┐     │
                                  │   Analyse data for new dataset│     │
                                  └──────────────────────────────┘     │
                                               │                       │
                                               ▼                       │
                                  ┌──────────────────────────────┐     │
                                  │    Establish correlations    │     │
                                  │    between objective and     │     │
                                  │   subjective information for │     │
                                  │         new dataset          │     │
                                  └──────────────────────────────┘     │
                                               │                       │
                                               ▼                       │
                                  ┌──────────────────────────────┐     │
                                  │   Compare dataset against    │     │
                                  │     previous dataset(s)      │     │
                                  └──────────────────────────────┘     │
                                               │                       │
                                               ▼                       │
                                  ┌──────────────────────────────┐     │
                                  │     Report back to user      │     │
                                  └──────────────────────────────┘     │
                                               │                       │
                                               ▼                       │
                                         ╱──────────╲      yes          │
                                        ╱ Behavioural ╲─────────────────┘
                                        ╲   change?   ╱
                                         ╲──────────╱
                                            no │
                                               ▼
                                  ┌──────────────────────────────┐
                                  │ Continue in mode I, II, III or V│
                                  └──────────────────────────────┘
```

FIG. 19

FIG. 20

FIG. 21

FIG. 22

| Monitor one or more objective parameters | ~ S2210 |

↓

| Collect data and communicate to processing means | ~ S2220 |

↓

| Collect objective test data using, using portable user interaction device | ~ S2230 |

↓

| Optional subjective feedback | ~ S2240 |

↓

| Process data to generate metric(s) | ~ S2250 |

↓

| Send information to portable user interaction device (8) information | ~ S2260 |

↓

| Repeat determination of metric(s) at later time | ~ S2270 |

Europäisches
Patentamt

European
Patent Office

Office européen
des brevets

## EUROPEAN SEARCH REPORT

**Application Number**

EP 10 16 9739

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/034289 A1 (TELLER ERIC [US] ET AL) 19 February 2004 (2004-02-19) * paragraph [0005] - paragraph [0009] * * page 0030 - page 0080 * ----- | 1-15 | INV. G06F19/00 |
| | | | TECHNICAL FIELDS SEARCHED (IPC) |
| | | | G06F |

The present search report has been drawn up for all claims

| Place of search | Date of completion of the search | Examiner |
|---|---|---|
| Munich | 9 November 2010 | Rinelli, Pietro |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   .......................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 10 16 9739

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2010

| Patent document cited in search report | Publication date | Patent family member(s) | | Publication date |
|---|---|---|---|---|
| US 2004034289 A1 | 19-02-2004 | AU | 2002330965 A1 | 24-02-2003 |
| | | BR | 0211760 A | 13-10-2004 |
| | | CA | 2454655 A1 | 20-02-2003 |
| | | EP | 1414340 A2 | 06-05-2004 |
| | | JP | 4283672 B2 | 24-06-2009 |
| | | JP | 2004538066 T | 24-12-2004 |
| | | MX | PA04001055 A | 20-05-2004 |
| | | WO | 03015005 A2 | 20-02-2003 |
| | | US | 2006122474 A1 | 08-06-2006 |
| | | US | 2007173705 A1 | 26-07-2007 |
| | | US | 2002019586 A1 | 14-02-2002 |

EPO FORM P0459

For more details about this annex : see Official Journal of the European Patent Office, No. 12/82

**EP 2 278 508 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6468234 B1
- US 20050042589 A1
- WO 2005066868 A2
- WO 2008096307 A1
- US 6743167 B2
- EP 1618913 A1
- US 7366572 B2
- US 20080157956 A1
- US 20060224047 A1
- US 5479939 A
- EP 1810710 A1, Junichiro Arai
- US 5229428 A, Mariko Kawaguri
- US 60218238 B, H. F. Machiel Van der Loos
- US 6485411 B2
- US 7179218 B2
- US 10718960 B
- US 7041049 B, Keith Raniere
- US 20040087878 A1, David T. Krausman
- US 20050096559 A1

- JP 2003369366 A, Kenichi Yanai
- US 20050143617 A1
- US 60534168 B
- US 20050222522 A1
- US 60553771 B
- US 20060135854 A9
- US 20060293602 A1
- US 20060293608 A1
- US 20070191692 A1
- US 20080033304 A1, Yousufali Dalal
- US 20040230398 A1
- US 2007008307 A1, Mi-hee Lee
- US 20080306351 A1
- WO 2004075714 A3
- WO 2005089641 A1
- WO 2004075714 A2, Cornel Lustig
- WO 2008037020 A1, Frances Renee Doherty
- US 20040225179 A1, Richard Kaplan
- US 7207938 B2

### Non-patent literature cited in the description

- **Dinges et al.** Cumulative sleepiness, mood disturbance and psychomotor vigilance performance decrements during a week of sleep restricted to 4-5 hours per night. *Sleep,* 1997, vol. 20, 267-277
- **Anthony Wetherell.** Cognitive and psychomotor performance tests and experiment design in multiple chemical sensitivity. *Environmental Health Perspectives,* March 1997, vol. 105 (2
- **Meir H. Kryger ; Thomas Roth ; William C. Dement.** Principles and practice of sleep medicine. Elsevier Saunders
- Socioeconomic impact of insomnia in working populations. *Industrial Health,* 2005, vol. 43, 11
- Evaluation of Quality of Life in Severe and Mild Insomniacs Compared With Good Sleepers. *Psychosomatic Medicine,* 2001, vol. 63, 49
- *Epidemiology of Insomnia, Depression, and Anxiety, Sleep,* 2005, vol. 11, 1457
- The Cost of Sleep-Related Accidents. *Sleep,* 1994, vol. 17, 84
- The Direct and Indirect Costs of Untreated Insomnia in Adults in the United States. *Sleep,* 2007, vol. 30, 263
- The economic cost of sleep disorders. *Sleep,* 2006, vol. 29, 299-305

- ABC of Sleep Disorders. BMJ. 1993
- **Murray W. Johns.** A new method for measuring daytime sleepiness: The Epworth sleepiness scale. *Sleep,* 1991, vol. 14 (6), 540-545
- **C. Janson et al.** Insomnia is more common among subjects living in damp buildings. *Occup Environ Med,* 2005, vol. 62, 113-118
- **John F. Schnelle ; Patrice A. Cruise ; Cathy A. Alessi ; Karen Ludlow ; Nahla R ; Al-Samarrai ; Joseph G. Ouslander.** Sleep Hygiene in Physically Dependent Nursing Home Residents: Behavioural and Environmental Intervention Implications. *Sleep,* 1998, vol. 21 (5), 515
- **G. J. Lavigne ; J. Y. Montplaisir.** Restless legs syndrome and sleep bruxism: prevalence and association among Canadians. *Sleep,* 1994, vol. 17 (8), 739-746
- The validity of psychomotor vigilance tasks of less than 10-minute duration. *Behaviour Methods, Instruments and Computers,* vol. 36 (2), 339-346
- Performance vigilance task and sleepiness in patients with sleep-disordered breathing. *European Respiratory Journal,* 2004, vol. 24, 279-285
- *Effects Of Sleep Deprivation On Performance: A Meta-Analysis, Sleep,* 1996, vol. 19, 318

- Cumulative sleepiness, mood disturbance and psychomotor vigilance performance decrements during a week of sleep restricted to 4-5 hours per night. *American Sleep Disorders Association and Sleep Research Society,* 1997, vol. 20 (4), 267-277
- **Okamoto-Mizuno K ; Mizuno K ; Michie S ; Maeda A ; Iizuka S.** Effects of humid heat exposure on human sleep stages and body temperature. *Sleep,* 1999, vol. 22, 767
- **E. H. Haskell et al.** The effects of high and low ambient temperatures on human sleep stages. *Electroencephalography and Clinical Neurophysiology,* 1981, vol. 51, 494-501
- **Patricia J. Murphy ; Scott S. Campbell.** Nighttime drop in body temperature: A physiological trigger for sleep onset?. *American Sleep Disorders Association and Sleep Research Society,* vol. 20 (7), 505-511
- **Franco P ; Szliwowski H ; Dramaix M ; Kahn A.** Influence of ambient temperature on sleep characteristics and autonomic nervous control in healthy infants. *Sleep,* 2000, vol. 23, 401
- **Franco P ; Scaillet S ; Valente Ir F ; Chabanski S ; Groswasser J ; Kahn A.** Ambient temperature is associated with changes in infants' arousability from sleep. *Sleep,* 2001, vol. 24, 325
- **Parmeggiani PL.** Interaction between sleep and thermoregulation: an aspect of the control of behavioural states. *Sleep,* 1987, vol. 10, 426
- **Libert JP ; Bach V ; Johnson LC ; Ehrhart J ; Wittersheim G ; Keller D.** Relative and combined effects of heat and noise exposure on sleep in humans. *Sleep,* 1991, vol. 14, 24

- **P. Franco ; H. Szliwowski ; M. Dramaix ; A. Khan.** Influence of ambient temperature on sleep characteristics and autonomic nervous control in healthy infants. *Sleep,* 2000, vol. 23, 401
- **J. P. Libert ; V. Bach ; L. C. Johnson ; J. Ehrhart ; G. Wittersheim ; D. Keller.** Relative and combined effects of heat and noise exposure on sleep in humans. *Sleep,* 1991, vol. 14 (1), 24-31
- **K I Hume ; F Van ; A Watson.** Effects of aircraft noise on sleep: EEG-based measurements. *Manchester Metropolitan University,* June 2003
- **J. A. Horne ; F. L. Pankhurst ; L. A. Reyner ; K. Hume ; I. D. Diamond.** A field study of sleep disturbances: Effects of aircraft noise and other factors on 5,742 nights of actimetrically monitored sleep in a large subject sample. *Sleep,* 1994, vol. 17 (2), 146-159
- **Yasuaki Nakagawa.** Sleep disturbances due to exposure to tone pulses throughout the night. *Sleep,* 1987, vol. 10 (5), 463-472
- **Joshua N. Aaron ; Carol C. Carlisle ; Mary A. Carskadon ; Thomas J. Meyer ; Nicholas S. Hill ; Richard P. Millman.** Environmental Noise As A Cause Of Sleep Disruption In An Intermediate Respiratory Care Unit. *Sleep,* 1996, vol. 19 (9), 707
- **N.L. Carter ; S.N. Hunyor ; G. Crawford ; D. Kelly ; A.J.M. Smith.** Environmental Noise And Sleep - A Study Of Arousals, Cardiac Arrhythmia And Urinary Catecholamines. *Sleep,* 1994, vol. 17 (4), 298